# EUROPEAN PATENT APPLICATION

(11) **EP 4 790 416 A2**
(43) Date of publication of application: **12.08.2026**
(21) Application number: 26157465.1
(22) Date of filing: 10.02.2026
(51) Int. Cl.: G01N 33/564, C07K 16/00, A61K 38/00

(54) **METHOD AND REAGENTS FOR THE DETECTION OF AN AUTOANTIBODY**

(30) Priority: 10.02.2025 EP 25156865; 01.08.2025 EP 25193331
(71) Applicant: EUROIMMUN Medizinische Labordiagnostika AG, 23560 Lübeck (DE); Hospices Civils de Lyon, 69002 Lyon (FR); Université Claude Bernard Lyon 1, 69100 Villeurbanne (FR); Centre National de la Recherche Scientifique, 75016 Paris (FR); Institut National de la Santé et de la Recherche Médicale, 75013 Paris (FR)
(72) Inventor: Miske, Ramona, 23560 Lübeck (DE); Scharf, Madeleine, 23560 Lübeck (DE); Müller, Yvonne, 23560 Lübeck (DE); Radzimski, Christiane, 23560 Lübeck (DE); Probst, Christian, 23560 Lübeck (DE); Komorowski, Lars, 23560 Lübeck (DE); Honnorat, Jérôme, 69002 Lyon (FR); Do, Le Duy, 69002 Lyon (FR); Pinto, Anne-Laurie, 69002 Lyon (FR)

(57) **Abstract**

The present invention relates to a method comprising the step detecting in a sample an antibody binding specifically to NECAB1, an antibody binding specifically to NECAB1, a carrier such as a diagnostically useful carrier with a solid phase with an immobilized polypeptide comprising an antigen or at least one epitope thereof or a variant of the antigen or epitope thereof, wherein the antigen is NECAB1, and a use of the antibody for diagnosing an autoimmune disease or a cancer.

## Description

The present invention relates to a method comprising the step detecting in a sample an antibody binding specifically to NECAB1, an antibody binding specifically to NECAB1, a carrier, preferably a diagnostically useful carrier, with a solid phase with an immobilized polypeptide comprising NECAB1 or at least one epitope thereof or a variant of NECAB1 or the epitope thereof and a use of the antibody for diagnosing a neurological autoimmune disease or a cancer.

Neurological autoimmune diseases are rare but are potentially treatable. Their hallmark is the occurrence of neurological symptoms and an autoimmune response, usually the emergence of an autoantibody binding specifically to structures in the brain such as polypeptides having important functions for neurotransmission. They can affect any area of the nervous system, including the central, peripheral and autonomic nervous system. Although the system involvement is often multifocal, like encephalomyelitis, it can involve a single system, e.g. cerebellar degeneration.

The neurological autoimmune disease may be associated with cancer. If that is the case, it is referred to as PNS (paraneoplastic syndrome). The neurological symptoms precede or follow the cancer diagnosis, although, in some cases, the primary cancer is not found even at autopsy. Most of the PNS reflect a nervous system-specific autoimmune attack initiated by onconeural antigens released to the peripheral lymphoid tissue from an unsuspected primary or recurrent neoplasm. Frequently, a cerebrospinal fluid (CSF) study in these patients reveals lymphocytic pleocytosis, elevated protein, increased IgG synthesis and oligoclonal bands, supporting the immunological pathology. Antibodies targeted against an accessible membrane target are directly responsible for the disease, as in the case of acetyl choline receptor antibodies in myasthenia gravis and P/Q type of voltage-gated calcium channels in Lambert Eaton Myasthenic syndrome. Often, the cancer is asymptomatic at the time of presentation with neurological syndrome. Some of the paraneoplastic antibodies are specifically associated with cancer and some are not.

Autoimmune encephalitis is another major neurological autoimmune disease, in particular anti-NMDA receptor autoimmune encephalitis which affects 1.5 patient per million per year. Symptoms such as paranoia, psychosis and violent behavior usually appear psychiatric in nature at first, but as the disease progresses, this may be accompanied by seizures, impaired cognition, memory deficit and speech problems. At a later stage treatment in an intensive care unit may be required if patients suffer from medically urgent symptoms including cerebellar ataxia, autonomic dysfunction and catatonia. An autoantibody to the NR1 subunit of the NMDA receptor was discovered as the cause (Dalmau et al., Anti-NMDA-receptor encephalitis: case series and analysis of the effects of antibodies, Lancet Neurology, Volume 7, Issue 12, 2008).

Early diagnosis and treatment of neurological autoimmune diseases is important because any delay can result in rapid progression and irreversible neurological damage. By contrast, early and appropriate treatment, usually by some form of immunosuppressive therapy, may reverse some of the damages. In some cases a complete recovery is possible. For example, 80% of patients suffering from NMDA receptor encephalitis have a good outcome with treatment, although long-term mental problems or a recurrence are possible.

Diagnosing neurological autoimmune diseases is often difficult, though. One of the reasons is the absence of a particular clinical pattern and absence of specific imaging and laboratory abnormalities. A combination of clinical and laboratory evaluations has to be deployed to reach diagnosis early. Treatment of underlying cancer, if present, is important in the treatment of the neurological condition.

Neurological autoantibodies are helpful for the diagnosis. Their presence, alone or in combination with other indicators, helps to establish the autoimmune nature of the disease, helping the clinician to differentiate the new neurological symptoms of a neurological autoimmune disease from a neurological condition which is the result of an infection, treatment-related-complications like toxic neuropathies drug abuse and psychiatric diseases. Moreover, they are of help in detecting the recurrence of the disease in already seropositive patients.

However, many patients suspected of suffering from a neurological autoimmune disease, 30 to 40% in the case of PNS, will not have any antibodies detectable by state of the art tests, not in the least because many diagnostically relevant antibodies are still unknown. There is the danger that these patients may be undiagnosed or even be misdiagnosed. In the past, some of them have been sent to psychiatric wards while, with appropriate immunosuppressive treatment, they could have lived reasonably normal lives.

The present invention is based on the inventors' surprising finding that antibodies to NECAB1 exist and can be detected in samples from patients suffering from a neurological autoimmune disease and/or cancer, but not in samples from healthy subjects. Hence, they can be used to aid in the diagnosis of diseases.

Therefore, the problem underlying the present invention is to provide reagents and methods for the diagnosis of a neurological autoimmune disease and/or cancer, preferably associated with the presence of an antibody binding specifically to NECAB1.

Another problem underlying the present invention is to distinguish an autoimmune disease caused by an antibody binding specifically to NECAB1 and a disease caused by other reasons, in particular an infection.

Another problem underlying the present invention is to provide an assay with an increased diagnostic reliability, in particular with regard to specificity and/or sensitivity, for the diagnosis for a neurological autoimmune disease or cancer, optionally in combination with state of the art assays.

The problem underlying the present invention is solved by the subject-matter of the attached independent and dependent claims.

In a first aspect, the problem underlying the present invention is solved by a method comprising the step detecting in a sample an antibody, preferably a mammalian autoantibody, binding specifically to an antigen, wherein the antigen is NECAB1, preferably according to SEQ ID NO1 or SEQ ID NO21.

In a second aspect, the problem underlying the present invention is solved by a method for isolating an antibody, preferably a mammalian autoantibody, binding specifically to an antigen, wherein the antigen is NECAB1, preferably according to SEQ ID NO1 or SEQ ID NO21, comprising the steps
a) Immobilizing on a carrier a polypeptide comprising the antigen or at least one epitope thereof or a variant of the antigen or epitope thereof,
b) contacting a sample comprising antibodies with the polypeptide under conditions compatible with formation of a complex, wherein said antibody binds to said polypeptide,
c) separating the complex formed in step a) from the sample,
d) optionally separating the antibody from the polypeptide, and
e) optionally detecting the antibody or complex.

In a third aspect, the problem underlying the present invention is solved by a method comprising the steps
a) immobilizing a polypeptide comprising an antigen or at least one epitope thereof or a variant of the antigen or epitope thereof on a carrier, wherein the antigen is NECAB1, preferably according to SEQ ID NO1 or SEQ ID NO21,
b) contacting the carrier with a liquid comprising an antibody, preferably mammalian autoantibody, binding specifically to the antigen, wherein a candidate drug is present in the liquid and/or the liquid does not comprise a sample from a subject to be diagnosed, preferably wherein the mammalian autoantibody is at a known concentration,
c) contacting the carrier with a means for detecting an immobilized antibody, and
d) detecting the presence, preferably in quantitative manner.

In a fourth aspect, the problem is solved by an antibody, preferably a mammalian autoantibody, binding specifically to an antigen, preferably in a liquid comprising one or more, more preferably all from the group comprising an artificial buffer, a preservative and an artificial anticoagulant, wherein the antigen is NECAB1, preferably according to SEQ ID NO1 or SEQ ID NO21.

In a fifth aspect, the problem is solved by a carrier, preferably a diagnostically useful carrier, with a solid phase with an immobilized polypeptide comprising an antigen I or at least one epitope thereof or a variant of the antigen I or epitope thereof and a) a negative control and/or b) at least one additional antigen II, preferably diagnostically useful antigen II, or at least one epitope thereof or variant of the antigen II or epitope thereof, wherein the polypeptide and the negative control or additional antigen II or epitope thereof or variant of the antigen II or epitope thereof are spatially separate on the carrier, or a first carrier, preferably a first diagnostically useful carrier, with a solid phase with an immobilized polypeptide comprising an antigen I or at least one epitope thereof or a variant of the antigen or epitope thereof and a second carrier, preferably a second diagnostically useful carrier, comprising a solid phase with an immobilized a) negative control and/or b) at least one additional polypeptide comprising at least one immobilized antigen II or at least one epitope thereof or a variant of the antigen II or epitope thereof, wherein the antigen I is NECAB1, preferably according to SEQ ID NO1 or SEQ ID NO21.

In a preferred embodiment, at least one additional antigen II is at least one antigen, preferably all antigens from the group consisting of NMDAR, Lgl1, AMPA1, AMPA2, CASPR2, GABA B, GABA A, DPPX, IGLON5, Hu, Yo, CV2/CRMP5, Ri, Ma2, Amphiphysin, Recoverin, RGS8, DAGLA, STX1B, AK5, AP3B2, Flotillin1+2, GRM1, GRM2, GRM5, GLURD2, ITPR1, KCNA2, NCDN, Septin 3+5+6+7+11 and Sez6L2.

In a sixth aspect, the problem is solved by a use of a) an antibody, preferably a mammalian autoantibody and/or a recombinant antibody, binding specifically to an antigen; or b) the combination of a polypeptide comprising an antigen or at least one epitope thereof or a variant of the antigen or epitope thereof and a means for detecting or capturing an IgG antibody; or c) a carrier comprising a polypeptide comprising an antigen or at least one epitope thereof or a variant of the antigen or epitope thereof for identifying a patient having an increased risk of developing a disease associated with the presence of an antibody binding specifically to the antigen and/or for diagnosing a neurological autoimmune disease or a cancer, wherein the antigen is NECAB1, preferably according to SEQ ID NO1 or SEQ ID NO21.

In a 7^{th} aspect, the problem is solved by a kit comprising a polypeptide comprising an antigen or at least one epitope thereof or a variant of the antigen or epitope thereof and a carrier, preferably a diagnostically useful carrier, more preferably the carrier according to the invention, wherein the antigen is NECAB1, preferably according to SEQ ID NO1 or SEQ ID NO21, wherein
a) the polypeptide is immobilized on a carrier, preferably a diagnostically useful carrier, more preferably the carrier according to the present invention, and the kit further comprises a means for detecting an immobilized antibody binding specifically to the antigen, preferably a secondary antibody specifically binding to human immunoglobulins, or
b) the polypeptide and the carrier are configured for immobilizing the polypeptide on the surface of the carrier, preferably *via* an affinity tag and a ligand binding to the affinity tag, and the kit further comprises a means for detecting an immobilized antibody, preferably a secondary antibody specifically binding to human immunoglobulins, or
c) the carrier is immobilized with a means for capturing an antibody, preferably a secondary antibody specifically binding to human immunoglobulins, and the kit further comprises a means for detecting a captured antibody, preferably the polypeptide comprising a detectable label, or
d) the carrier and a means for capturing an antibody, preferably a secondary antibody specifically binding to human immunoglobulins, are configured for immobilizing the means for capturing an antibody on the surface of the carrier, preferably via an affinity tag and a ligand binding to the affinity tag, and the kit further comprises a means for detecting an immobilized antibody, preferably the polypeptide comprising a detectable label,
and preferably one or more, more preferably all from the group consisting of a recombinant antibody binding specifically to the antigen, an isolated antibody, preferably mammalian autoantibody, binding specifically to the antigen, a chemical solution reactive with a detectable label, a positive control, a negative control, a water-tight vessel for incubating a sample with a carrier or reagent, a wash buffer, and a calibrator, preferably a set of calibrators.

In a preferred embodiment, the kit comprises a secondary antibody and/or a polypeptide comprising NECAB1 or an epitope thereof or a variant of NECAB1 or the epitope thereof, optionally wherein the secondary antibody and/or the polypeptide are labeled.

In an 8^{th} aspect, the problem is solved by use of a polypeptide comprising an antigen or at least one epitope thereof or a variant of the antigen or epitope thereof or an antibody, preferably autoantibody, binding specifically to the antigen or a recombinant antibody binding specifically to the antigen for the manufacture of a kit or medical device, preferably diagnostic device, preferably for diagnosing or aiding in the diagnosis of a disease, preferably associated with the presence of a mammalian antibody, preferably autoantibody, binding specifically to NECAB1, more preferably a neurological autoimmune disease and/or a cancer, wherein the antigen is NECAB1, preferably according to SEQ ID NO1 or SEQ ID NO21.

In a 9^{th} aspect, the problem is solved by a use of an antibody, preferably a mammalian autoantibody, binding specifically to an antigen or a recombinant antibody binding specifically to the antigen, which is preferably recognized by secondary antibodies to human immunoglobulins, preferably of class IgG, as a positive control for the detection of an antibody, preferably mammalian autoantibody, binding specifically to the antigen in a sample, wherein the antigen is NECAB1, preferably according to SEQ ID NO1 or SEQ ID NO21.

In a 10^{th} aspect, the problem is solved by an *ex vivo* method for removing an antibody, preferably a mammalian autoantibody, binding specifically to an antigen from a bodily fluid, preferably blood, preferably serum, or CSF of a patient, wherein the antigen is NECAB1, preferably according to SEQ ID NO1 or SEQ ID NO21.

In an 11^{th} aspect, the problem is solved by a method for diagnosing or aiding in the diagnosis of a disease, preferably associated with the presence of a mammalian antibody, preferably autoantibody, binding specifically to NECAB1, such as an autoimmune disease, associated with an antibody, preferably a mammalian autoantibody, binding specifically to NECAB1, preferably according to SEQ ID NO1 or SEQ ID NO21. In one embodiment, the method for diagnosing a disease is an *in vivo* method. In one embodiment, the method for diagnosing a disease is an *ex vivo* method. In one embodiment, the method for diagnosing a disease comprises using a polypeptide comprising an antigen or at least one epitope thereof or a variant of the antigen or epitope thereof, wherein the antigen is NECAB1, preferably according to SEQ ID NO1 or SEQ ID NO21. In one embodiment, the disease is an autoimmune disease, preferably a neurological autoimmune disease, such as those described herein, and/or a cancer, such as those described herein. In one embodiment, the method for diagnosing a disease comprises the step detecting an antibody, preferably autoantibody, binding specifically to an antigen from blood, preferably serum of a patient, wherein the antigen is NECAB1, preferably according to SEQ ID NO1 or SEQ ID NO21.

In a 12^{th} aspect, the problem is solved by a kit comprising a polypeptide comprising NECAB1 or an epitope thereof or a variant of NECAB1 or the epitope thereof and a means for detecting an antibody and/or a means for capturing an antibody. In one embodiment, the polypeptide may comprise a label. In one embodiment, the kit comprises a secondary antibody specifically binding to mammalian, preferably human immunoglobulins. In one embodiment, the secondary antibody may comprise a label.

In a preferred embodiment, the disease associated with the presence of a mammalian antibody, preferably autoantibody, binding specifically to NECAB1, preferably the autoimmune disease is a neurological autoimmune disease associated with the presence of an antibody, preferably a mammalian autoantibody, specifically binding to NECAB1, preferably selected from the group consisting of paraneoplastic neurological syndrome (PNS), dementia, psychotic symptoms, acoustic hallucinations, seizures, encephalitis, such as limbic encephalitis, brainstem encephalitis or autoimmune encephalitis, peripheral polyneuropathy, Guillain-Barré syndrome (GBS) symptoms, tetraparesis, cerebellar/brainstem syndrome, hearing loss, symmetric maculopathy, cerebellopontine lesion extending into spinal cord, personality changes, cognitive impairment and affect disorder, gait impairment, gait and stand ataxia, multiple cerebral ischemia, and/or cancers such as carcinoma, cerebral metastases, mamma carcinoma, lung cancer, such as lung carcinoma, and melanoma. In another preferred embodiment, the autoimmune disease is a neurological autoimmune disease associated with the presence of an antibody specifically binding to NECAB1, preferably selected from the group consisting of paraneoplastic neurological syndrome (PNS), personality changes, cognitive impairment and affect disorder, gait impairment, encephalopathy, encephalomyelitis, myelitis, cerebellitis, neuropathy, dementia, encephalitis, such as limbic encephalitis, brainstem encephalitis or autoimmune encephalitis, neuronopathy, cerebellar/brainstem syndrome and/or a cancer such as mamma carcinoma and lung cancer, such as lung carcinoma.

In a preferred embodiment, the polypeptide is a recombinant, isolated and/or purified polypeptide.

In a preferred embodiment, the antigen the antibody binds to is NECAB1.

In a preferred embodiment, the antibody is a mammalian, preferably human antibody and/or the sample is a mammalian, preferably human sample comprising a representative set of antibodies, preferably selected from the group comprising whole blood, plasma, serum, cerebrospinal fluid and saliva. In a preferred embodiment, the antibody is an autoantibody. In a preferred embodiment, the antibody binds specifically to the antigen. In a preferred embodiment, the antibody comprises a human Fc region. In a preferred embodiment, the antibody, preferably the autoantibody, comprises one or more sequences selected from the group comprising SEQ ID NO24, SEQ ID NO25, SEQ ID NO26, SEQ ID NO27, SEQ ID NO28, SEQ ID NO29, SEQ ID NO30, SEQ ID NO31, SEQ ID NO32, SEQ ID NO33, SEQ ID NO34, SEQ ID NO35, SEQ ID NO36, SEQ ID NO37 and SEQ ID NO38, preferably selected from the group comprising SEQ ID NO28, SEQ ID NO29, SEQ ID NO30 and SEQ ID NO31. In one embodiment, the antibody is isolated. In one embodiment, the antibody is in a blood sample, such as whole blood, wherein the blood sample is optionally diluted.

In a preferred embodiment, the antibody or complex is detected using a detection method selected from the group comprising immunodiffusion, immunoelectrophoresis, light scattering immunoassays, agglutination, labeled immunoassays such as those from the group comprising radiolabeled immunoassay, enzyme immunoassays, more preferably ELISA, chemiluminescence immunoassays, preferably electrochemiluminescence immunoassay, and immunofluorescence, preferably indirect immunofluorescence.

In a preferred embodiment, the carrier is selected from the group comprising a glass slide, preferably for microscopy, a biochip, a microtiter plate, a lateral flow device, a test strip, a membrane, preferably a line blot, a chromatography column and a bead, preferably a magnetic or fluorescent bead.

The present invention is based on the inventors' surprising finding that antibodies to NECAB1, preferably according to SEQ ID NO1 or SEQ ID NO21, exist and can be detected in samples from patients suffering from a neurological autoimmune disease and/or cancer, but not in samples from healthy subjects. Hence, they can be used to aid in the diagnosis of diseases, such as autoimmune diseases, for example a neurological autoimmune disease as described herein, and/or in differentiating between a neurological autoimmune disease and a neurological disease of different etiology but with similar symptoms such as an infectious neurological disease or an aseptic non-autoimmune neurological disease, for example between autoimmune encephalitis, including limbic encephalitis and brainstem encephalitis, and infectious and/or aseptic non-autoimmune encephalitis or meningitis, including encephalitis of infectious etiology, such as viral encephalitis, bacterial encephalitis, Tick-born encephalitis, Herpes encephalitis, CMV encephalitis, Toxoplasmic encephalitis, meningitis of infectious etiology, such as viral meningitis, bacterial meningitis, and aseptic meningitis and encephalitis not associated with an autoantibody, in particular binding specifically to NECAB1.

SEQ ID NO1 represents the sequence of NECAB1, the N-terminal EF-hand calcium-binding protein 1. Said protein has the uniprot database reference UniProtKB - Q8N987 (NECA1_HUMAN) and has calcium ion binding activity. It is involved in diverse biological processes, such as blastocyst hatching, regulation of amyloid precursor protein biosynthetic process and others. One isoform of the protein is known (uniprot reference Q8N987-2, SEQ ID NO22). Thus, in one embodiment, NECAB1 comprises SEQ ID NO1 or a variant thereof. In one embodiment, NECAB1 comprises one or more sequences selected from the group comprising SEQ ID NO18, SEQ ID NO19, SEQ ID NO20, SEQ ID NO21, SEQ ID NO22 and SEQ ID NO23 or a variant thereof. In a preferred embodiment, NECAB1 is selected from or comprises a sequence selected from the group comprising SEQ ID NO1, SEQ ID NO18, SEQ ID NO19, SEQ ID NO20, SEQ ID NO21, SEQ ID NO22 and SEQ ID NO23 or a variant thereof, more preferably from the group comprising SEQ ID NO1, SEQ ID NO18, SEQ ID NO19 and SEQ ID NO20.

In preferred embodiments, the antigen used in the methods of the invention comprises NECAB1 and/or a variant thereof. The variant of NECAB1 may be selected from the group comprising SEQ ID NO2, SEQ ID NO3, SEQ ID NO4, SEQ ID NO5, SEQ ID NO6, SEQ ID NO7, SEQ ID NO8, SEQ ID NO9, SEQ ID NO10, SEQ ID NO18, SEQ ID NO19, SEQ ID NO20, SQE ID NO21, SEQ ID NO22 and SEQ ID NO23 or a variant thereof, preferably comprising SEQ ID NO18, SEQ ID NO19, SEQ ID NO20, SEQ ID NO21, SEQ ID NO22 and SEQ ID NO23 or a variant thereof, even more preferably from the group comprising SEQ ID NO18, SEQ ID NO19, SEQ ID NO20 and SEQ ID NO22, even more preferably from the group comprising SEQ ID NO18, SEQ ID NO19 and SEQ ID NO20 or a variant thereof. In other preferred embodiments, an antibody, preferably autoantibody, specifically binding to NECAB1 is associated with a neurological autoimmune disease and/or a cancer, preferably selected from the group comprising personality changes, cognitive impairment and affect disorder, mamma carcinoma, lung cancer, such as lung carcinoma, PNS, encephalitis, such as limbic encephalitis, brainstem encephalitis or autoimmune encephalitis, neuropathy, neuronopathy, cerebellitis, myelitis, and/or cerebellar syndrome.

In a preferred embodiment, the antigen of the invention is from a mammal selected from the group comprising a human, a non-human primate, a rodent, a cow, a horse, a dog, a cat, a bear, a donkey, a sheep, a goat, a camel and a dromedary, more preferably a human. In another preferred embodiment, the antigen of the invention comprises at least one polypeptide comprising at least one sequence selected from the group comprising SEQ ID NO1, SEQ ID NO2, SEQ ID NO3, SEQ ID NO4, SEQ ID NO5, SEQ ID NO6, SEQ ID NO7, SEQ ID NO8, SEQ ID NO9, SEQ ID NO10, SEQ ID NO18, SEQ ID NO19, SEQ ID NO20, SEQ ID NO21, SEQ ID NO22 and SEQ ID NO23 or a variant thereof, preferably from the group comprising SEQ ID NO18, SEQ ID NO19, SEQ ID NO20 and SEQ ID NO22 or a variant thereof and, optionally, having a length of at least 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 380, 400, 420, 440, 450, 460, 480, 500, 520, 540, 550, 560, 580, 600, 620, 640, 650, 660, 680, 700, 720, 740, 750, 760, 780, 800, 820, 840, 850, 860, 880, 900, 920, 940, 950, 960, 980, 1000, 1050, 1100, 1150, 1200, 1250 or more amino acids.

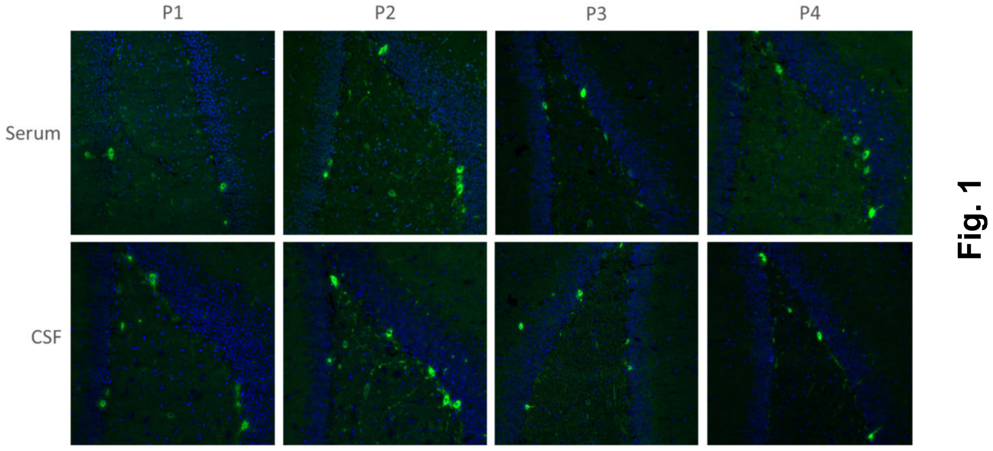

In a preferred embodiment, the method according to the present invention comprises the step providing the carrier according to the present invention. A polypeptide comprising an antigen such as NECAB1 or at least one epitope thereof or a variant of the antigen or epitope thereof may be immobilized on a solid surface of the carrier or the means may be configured for immobilizing a transporter comprising the antigen on the carrier, which may be done at a later stage, but before removing the sample and washing the carrier. The carrier may then be contacted with the sample suspected of comprising the antibody under conditions allowing for binding of any antibodies to NECAB1. The sample may then be removed and the carrier may be washed to remove any remaining sample. A means for detecting the antibody carrying a detectable label such as a fluorescent dye may then be contacted with the carrier under conditions allowing formation of a complex between any bound antibody and the means. The carrier may be washed again. Finally, the presence of the antibody is detected by checking whether the means such as a secondary antibody may be detected. Preferably an ELISA or an immunofluorescence assay using a mammalian cell or tissue expressing a polypeptide comprising the antigen of the invention or a variant thereof is used. In the case of immunofluorescence, a distinct pattern determined by the expression pattern of the antigen in the cell or the tissue indicates the presence of the antibody, as carried out or shown in the examples (Figure 1). In the case of an ELISA or another semi-quantitative or quantitative detection method a value above a cut off value determined as known in the art indicates the presence of the antibody.

A competitive assay, a capture bridge assay, an immunometric assay, a class-specific second antibody on the solid phase, a direct or indirect class capture assay may also be used. The principle of each of these formats is detailed in The Immunoassay Handbook, 3rd edition, edited by David Wild, Elsevier, 2005. Briefly, in a competitive format, the antibody to be detected may compete with a recombinant antibody for binding sites, such as epitopes, on the antigen, which is NECAB1 or a variant thereof. Alternatively, a sample comprising the antibody may be preincubated with the antigen, followed by exposure to immobilized antigen or antigen configured for immobilization in one reaction and may be exposed to the antigen without preincubation with the antigen in another reaction to show specific binding. In a capture bridge assay, two antigen molecules bind to two antigen binding sites on the antibody to be detected. One of the antigen molecules is labeled and the other one immobilized or configured for immobilization, preferably via an affinity tag and a ligand binding specifically to said affinity tag. In an immunometric assay, the antibody to be detected binds to the antigen, which is immobilized after or before the binding. The antibody is detected using a means for detecting an antibody such as a labeled secondary antibody. In a direct class capture assay, the antibody to be detected is immobilized using a means for capturing an antibody and detected using a labeled antigen. In an indirect class capture assay, the antibody to be detected is immobilized using a means for capturing an antibody. It is detected using at least one, preferably two molecules of the antigen which bind to the antibody to be detected, and a means for detecting an antibody such as a labeled secondary antibody. Instead of the antigen, polypeptide(s) comprising at least one epitope of the said antigen or a variant of the antigen or epitope thereof can be used in any of the aforementioned assays.

In a preferred embodiment, the carrier and a means for capturing and/or a means for detecting an antibody are provided, preferably wherein the carrier and the means for capturing and/or the means for detecting are configured for immobilizing the means on the solid surface of the carrier. A particularly preferred way for the configuration or the immobilization is modifying the carrier and/or the means such that they comprise an affinity tag and a ligand to the affinity tag. Alternatively, the carrier or the means may comprise reactive chemical groups such as thiol, amino, epoxide, ester and anhydride groups. In a preferred embodiment, the term "immobilized", as used herein, refers to a molecule bound to a solid carrier insoluble in an aqueous solution, more preferably via a covalent or non-covalent bond, electrostatic interactions, encapsulation, unspecific absorption, printing or entrapment, for example by denaturing a globular polypeptide in a gel, or via hydrophobic interactions, most preferably via one or more covalent bonds. Various suitable carriers, for example paper, polystyrene, metal, silicon or glass surfaces, microfluidic channels, membranes, beads such as magnetic beads, column chromatography media, biochips, polyacrylamide gels and the like have been described in the literature, for example in Kim, D., and Herr, A. E. (2013), Protein immobilization techniques for microfluidic assays, Biomicrofluidics 7(4), 041501. This way, the immobilized molecule, together with the insoluble carrier, may be separated from an aqueous solution in a straightforward manner, for example by centrifugation or decanting. An immobilized molecule may be immobilized in a reversible or irreversible manner. For example, the immobilization is reversible if the molecule interacts with the carrier via ionic interactions that can be masked by addition of a high concentration of salt or if the molecule is bound via a cleavable covalent bond such as a disulfide bridge which may be cleaved by addition of thiol-containing reagents. By contrast, the immobilization is irreversible if the molecule is tethered to the carrier via a covalent bond that cannot be cleaved in aqueous solution, for example a bond formed by reaction of an epoxide group and an amine group as frequently used to couple lysine side chains to affinity columns. The protein may be indirectly immobilized, for example by immobilizing an antibody or other entity having affinity to the molecule, followed by formation of a complex to the effect that the molecule-antibody complex is immobilized. Various ways to immobilize molecules are described in the literature, for example in Kim, D., and Herr, A. E. (2013), Protein immobilization techniques for microfluidic assays, Biomicrofluidics 7(4), 041501. In addition, various reagents and kits for immobilization reactions are commercially available, for example from Pierce Biotechnology.

According to the present invention, a cell may be provided, which comprises an expression vector comprising a nucleotide sequence encoding a polypeptide comprising an antigen or at least one epitope thereof or a variant of the antigen or epitope thereof, wherein the antigen is NECAB1 or a variant thereof, under the control of a promotor, optionally a strong and/or inducible promotor. The vector may encode for an N-terminal or C-terminal affinity tag fused to the polypeptide, preferably via a linker sequence. The cell may be cultured under conditions allowing for the expression of the antigen of the invention. Any expressed antigen may then be purified, preferably using the affinity tag. However, a non-purified antigen may also be used in some embodiments. Subsequently it is immobilized on the carrier according to the present invention. In a preferred embodiment, the cell, nucleic acid or vector may be used for the manufacture of a kit for the diagnosis of a neurological autoimmune disease.

According to the present invention, a medical or diagnostic device such as the carrier, preferably diagnostically useful carrier, of the invention may be prepared by expressing a recombinant polypeptide comprising NECAB1 or at least one epitope thereof or a variant of NECAB1 or the epitope thereof comprising an affinity tag, optionally with an artificial linker, which may include a protease cleavage site, in a cell such as a eukaryotic or prokaryotic cell. Contacting the polypeptide with a ligand binding specifically to the affinity tag, which ligand is immobilized on a solid phase, washing the solid phase such that non-specifically bound material from the cell is removed and eluting the expressed variant from the solid phase, preferably by adding an excess of non-immobilized ligand. The variant may then be immobilized on the device. Optionally, the affinity tag may be removed by contacting the variant with a protease, preferably a protease recognizing the protease cleavage site, before the immobilization. The affinity tag may be selected from the group of tags comprising His, immobilized nickel, glutathione, chitin, 18A, ACP, Aldehyd, Avi, BCCP, Calmodulin, Chitin binding protein, E-Tag, ELK16, FLAG, flash, poly glutamate, poly aspartate, GST, GFP, HA, Isope, maltose binding protein, myc, nus, NE, ProtA, ProtC, Tho1d4, S-Tag, SnoopTag, SpyTag, SofTag, Streptavidin, Strep-tag II, T7 Epitope Tag, TAP, TC, Thioredoxin, Ty, V5, VSV, biotin, Xpress Tag and a recombinant antibody binding to the ligand to an affinity tag. Useful proteases include, but are not limited to TEV, Thrombin, Faktor Xa or Enteropeptidase. Suitable linkers are part of vectors, for example pET vector series (Novagen).

In a preferred embodiment, the presence or absence of at least one antibody other than an antibody binding specifically to an antigen of the invention is detected, preferably from the group consisting of an autoantibody binding specifically to NMDAR, an autoantibody binding specifically to Lg11, an autoantibody binding specifically to AMPA1, an autoantibody binding specifically to AMPA2, an autoantibody binding specifically to CASPR2, an autoantibody binding specifically to GABA B, an autoantibody binding specifically to GABA A, an autoantibody binding specifically to DPPX, an autoantibody binding specifically to IGLON5, an autoantibody binding specifically to Hu, an autoantibody binding specifically to Yo, an autoantibody binding specifically to CRMP5, an autoantibody binding specifically to Ri, an autoantibody binding specifically to Ma2, an autoantibody binding specifically to Amphiphysin, an autoantibody binding specifically to Recoverin, an autoantibody binding specifically to RGS8, an autoantibody binding specifically to DAGLA, an autoantibody binding specifically to NSF, an autoantibody binding specifically to STX1B, an autoantibody binding specifically to DNM1, an autoantibody binding specifically to VAMP2, an autoantibody binding specifically to Anna-3, an autoantibody binding specifically to Zic-4, an autoantibody binding specifically to SOX1 (US7314721), an autoantibody binding specifically to PCA2, an autoantibody binding specifically to Tr, an autoantibody binding specifically to glutamic acid decarboxylase, an autoantibody binding specifically to AK5, an autoantibody binding specifically to AP3B2, an autoantibody binding specifically to Flotillin1/2, an autoantibody binding specifically to GRM1, an autoantibody binding specifically to GRM2, an autoantibody binding specifically to GRM5, an autoantibody binding specifically to GLURD2, an autoantibody binding specifically to ITPR1, an autoantibody binding specifically to KCNA2, an autoantibody binding specifically to NCDN, an autoantibody binding specifically to Septin 3+5+6+7+11 and an autoantibody binding specifically to Sez6L2. In a preferred embodiment, the detection of the presence of any of these autoantibodies aid in the diagnosis or implies a diagnosis of an autoimmune disease, such as a neurological autoimmune disease. In a preferred embodiment, two or more autoantibodies are detected in the same sample and preferably essentially simultaneously. The carrier according to the present invention may be configured for detecting two or more autoantibodies in the same sample, preferably essentially simultaneously.

In a preferred embodiment, the presence or absence of two or more antibodies is detected and can be distinguished. In other words, a signal indicating the presence of an antibody indicates which of the two antibodies is present. In a preferred embodiment, the absence or presence of two or more antibodies is detected in spatially separate reactions, more preferably in different reaction mixtures in separate vessels. In another preferred embodiment, a signal indicating the presence of one of the two antibodies may be distinguished from a signal indicating the presence of the other antibody. This may be achieved by using different detectable labels, more preferably distinguishable fluorophores. For example, a fluorophore emitting green light and another one emitting red light may be used.

In a preferred embodiment, the presence or absence of two or more antibodies is detected, but cannot be distinguished. In a preferred embodiment, their absence or presence is detected in a one pot reaction, preferably in two or more reactions in the same reaction vessel without spatial separation, and with no signal distinction. In other words, a signal indicates that at least one of the two antibodies is present, but not which one. In a preferred embodiment, two or more antigens may be present in a mixture.

In a preferred embodiment, the sample comprises a representative set of antibodies, more preferably IgG, IgA and IgM class antibodies, most preferably IgG class antibodies. It is preferably selected from the group consisting of whole blood, plasma, serum, cerebrospinal fluid (CSF) and saliva. The sample may be a liquid sample or a dried blood spot made using the sample, preferably whole blood, plasma, serum or capillary blood, preferably capillary blood.

In a preferred embodiment, the sample is from an organism having a brain and producing antibodies, preferably from the group comprising mammals and birds, more preferably a mammal from the group consisting of a human, a non-human primate, a rodent (such as a rat or mouse), a cow, a horse, a dog, a cat, a bear, a donkey, a sheep, a goat, a camel and a dromedary, most preferably a human. In another preferred embodiment it is from a bird, more preferably from the group comprising a chicken, a parrot and a falcon. The animal may have gone through extensive training, for example for assisting people in need, for riding or for hunting.

The antibody to be detected or a means for detecting or capturing an antibody binds specifically to its interaction partner, which is an antigen in the case of an antibody or an antibody in the case of a means for detecting an antibody, preferably the antibody binding specifically to NECAB1 or a variant thereof. In a preferred embodiment, the term "binding specifically", as used herein, means that the binding reaction is stronger than a binding reaction characterized by a dissociation constant of 1 x 10⁻⁵ M, more preferably 1 x 10⁻⁷ M, more preferably 1 x 10⁻⁸ M, more preferably 1 x 10⁻⁹ M, more preferably 1 x 10⁻¹¹ M, more preferably 1 x 10⁻¹¹ M, more preferably 1 x 10⁻¹² M, as determined by surface plasmon resonance using Biacore equipment at 25 °C in PBS buffer at pH 7.

In a preferred embodiment, the term "autoantibody", as used herein, refers to an antibody which binds specifically to a structure, preferably an antigen, more preferably at least one epitope of said antigen, from the organism which produces said antibody. The organism is preferably a patient suspected of or actually suffering from a disease, preferably a mammalian, more preferably human patient. Such an autoantibody has a constant region, as have other antibodies of the same class from the same organism. Particularly preferably, the autoantibody is a mammalian autoantibody, even more preferably a human autoantibody, even more preferably a human autoantibody of class IgG, IgM or IgA, preferably IgG. The variable domain is capable of binding specifically against the antigen. The constant domain binds specifically to molecules recognizing the constant domain of the immunoglobulin class of the antibody, preferably IgG class antibodies, such as secondary antibodies. It has sequence elements shared by other antibodies, preferably IgG class antibodies from the same organism.

According to the present invention, an antibody binding specifically to NECAB1 or a variant thereof is provided or isolated or used. The person skilled in the art is familiar with the isolation or purification of antibodies. Comprehensive instructions are avaliable in the prior art, for example in Affinity Chromatography Vol. 1 Antibody by GE Healthcare, www.gelifesciences.com, April 2016 and Thermo Scientific Pierce Antibody Production and Purification Technical Handbook, Version 2, www.thermoscientific.com. For example, specific purification steps may involve affinity chromatography using a polypeptide comprising the antigen of the invention or a variant thereof immobilized to beads by coupling using primary amines and/or Protein G.

In a preferred embodiment, a subject or a patient as used herein is a mammalian, preferably a human subject or a mammalian, preferably a human patient. In a preferred embodiment, a patient is a subject having or suspected of having a disease associated with the presence of a mammalian antibody binding specifically to NECAB1.

The teachings of the present invention may not only be carried out using the polypeptides, in particular a polypeptide comprising the native sequence of a polypeptide referred to such as NECAB1 or a variant thereof or nucleic acids having the exact sequences referred to in this application explicitly, for example by function, name, sequence or accession number, or implicitly, but also using variants of such polypeptides or nucleic acids.

In a preferred embodiment, the term "variant", as used herein, may refer to at least one fragment of the full length sequence referred to, more specifically one or more amino acid or nucleic acid sequence which is, relative to the full-length sequence, truncated at one or both termini by one or more amino acids. Such a fragment comprises or encodes for a peptide having at least 6, 7, 8, 10, 12, 15, 20, 25, 30, 40, 50, 60, 70, 75, 80, 90, 100, 110, 120, 130, 140, 150, 160, 170, 180, 190, 200, 210, 220, 230, 240, 250, 260, 270, 280, 290, 300, 310, 320, 330, 340, 350, 360, 380, 400, 450, 500, 600, 620, 640, or 660 successive amino acids of the original sequence or a variant thereof. The total length of the variant may be at least 6, 7, 8, 9, 10, 11, 12, 20, 25, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 380, 400, 450, 500, 520, 540, 560, 580, 600, 610, 620, 630, 640, 650, 660 or 670 or more amino acids.

The term "variant" relates not only to at least one fragment, but also to a polypeptide or a fragment thereof comprising amino acid sequences that are at least 40, 50, 60, 70, 75, 80, 85, 90, 92, 94, 95, 96, 97, 98, 99, 99,3, 99,4, 99,5, 99,6, 99,7, 99,8 or 99,9, preferably at least 80% or 99% identical to the reference amino acid sequence referred to or the fragment thereof, preferably wherein amino acids other than those essential for the biological activity, for example the ability of an antigen to bind to an antibody, or the fold or structure of the polypeptide are deleted or substituted and/or one or more such essential amino acids are replaced in a conservative manner and/or amino acids are added such that the biological activity of the polypeptide is preserved. In other words, in preferred embodiments the variant maintains the biological activity (of the full length protein of which it is derived). The state of the art comprises various methods that may be used to align two given nucleic acid or amino acid sequences and to calculate the degree of identity, see for example Arthur Lesk (2008), Introduction to bioinformatics, Oxford University Press, 2008, 3rd edition. In a preferred embodiment, the ClustalW software (Larkin, M. A., Blackshields, G., Brown, N. P., Chenna, R., McGettigan, P. A., McWilliam, H., Valentin, F., Wallace, I. M., Wilm, A., Lopez, R., Thompson, J. D., Gibson, T. J., Higgins, D. G. (2007). Clustal W and Clustal X version 2.0. Bioinformatics, 23, 2947-2948) is used using default setting.

In a preferred embodiment, the polypeptide and variants thereof may comprise a tag, such as an affinity tag, wherein the tag preferably is selected from the group comprising His tag, GST tag, E tag, FLAG tag, HA tag, myc tag, V5 tag, S tag, SnoopTag, SpyTag, SofTag, Strep tag, Strep tag II, T7 Epitope tag, biotin tag, ALFA-tag, AviTag, C-Tag, calmodulin tag, iCap tag, polyglutamate tag, polyarginine tag, NE-tag, Rho1D4-tag, SBP-tag, Softag 1, Softag 3, Spot-tag, T7-tag, TC tag, Ty tag, VSV-tag, and Xpress tag.

In a preferred embodiment, the polypeptide and variants thereof may, in addition, comprise chemical modifications, for example isotopic labels or covalent modifications such as glycosylation, phosphorylation, acetylation, decarboxylation, citrullination, methylation, hydroxylation, ubiquitylation and the like. The person skilled in the art is familiar with methods to modify polypeptides. Any modification is designed such that it does not abolish the biological activity of the antigen or epitope of the invention or the variant.

Moreover, variants may also be generated by N- and/or C-terminal fusion of polypeptides, fragments or variants thereof with other known polypeptides or variants thereof or artificial sequences such as linkers and comprise active portions or domains, preferably having a sequence identity of at least 70, 75, 80, 85, 90, 92, 94, 95, 96, 97, 98 or 99% when aligned with the active portion of the reference sequence, wherein the term "active portion", as used herein, refers to an amino acid sequence, which is less than the full length amino acid sequence or, in the case of a nucleic acid sequence, codes for less than the full length amino acid sequence, respectively, and/or is a variant of the natural sequence, but retains at least some of the biological activity. Preferably the active portion is an active portion of NECAB1. The polypeptide may comprise additional sequences, preferably artificial sequences for example linkers or binding epitopes. Any fused sequences are chosen such that the ability of the antigen of the invention or a variant thereof to bind specifically to the antibody to be detected or the diagnostic reliability, in particular sensitivity and/or specificity, is not significantly altered, let alone abolished.

In a preferred embodiment, the term "variant" of a nucleic acid comprises nucleic acids the complementary strand of which hybridizes, preferably under stringent conditions, to the reference or wild type nucleic acid. Stringency of hybridization reactions is readily determinable by one of ordinary skill in the art, and in general is an empirical calculation dependent on probe length, washing temperature and salt concentration. In general, longer probes require higher temperatures for proper annealing, while shorter probes less so. Hybridization generally depends on the ability of denatured DNA to reanneal to complementary strands present in an environment below their melting temperature: The higher the degree of desired homology between the probe and hybridizable sequence, the higher the relative temperature which may be used. As a result, it follows that higher relative temperatures would tend to make the reaction conditions more stringent, while lower temperatures less so. For additional details and explanation of stringency of hybridization reactions, see Ausubel, F. M. (1995), Current Protocols in Molecular Biology. John Wiley & Sons, Inc. Moreover, the person skilled in the art may follow the instructions given in the manual Boehringer Mannheim GmbH (1993) The DIG System Users Guide for Filter Hybridization, Boehringer Mannheim GmbH, Mannheim, Germany and in Liebl, W., Ehrmann, M., Ludwig, W., and Schleifer, K. H. (1991) International Journal of Systematic Bacteriology 41: 255-260 on how to identify DNA sequences by means of hybridization. In a preferred embodiment, stringent conditions are applied for any hybridization, i.e. hybridization occurs only if the probe is 70% or more identical to the target sequence. Probes having a lower degree of identity with respect to the target sequence may hybridize, but such hybrids are unstable and will be removed in a washing step under stringent conditions, for example lowering the concentration of salt to 2 x SSC or, optionally and subsequently, to 0,5 x SSC, while the temperature is, in order of increasing preference, approximately 50°C - 68°C, approximately 52°C - 68°C, approximately 54°C - 68°C, approximately 56°C - 68°C, approximately 58°C - 68°C, approximately 60°C - 68°C, approximately 62°C - 68°C, approximately 64°C - 68°C, approximately 66°C - 68°C. In a particularly preferred embodiment, the temperature is approximately 64°C - 68°C or approximately 66°C - 68°C. It is possible to adjust the concentration of salt to 0.2 x SSC or even 0.1 x SSC. Nucleic acid sequences having a degree of identity with respect to the reference or wild type sequence of at least 70, 80, 90, 91, 92, 93, 94, 95, 96, 97, 98, 99% may be isolated. In a preferred embodiment, the term variant of a nucleic acid sequence, as used herein, refers to any nucleic acid sequence that encodes the same amino acid sequence, preferably NECAB1 or a variant thereof, as the reference nucleic acid sequence, in line with the degeneracy of the genetic code.

The polypeptide used to carry out the inventive teachings, including any variants, is preferably designed such that it comprises at least one epitope recognized by and/or binds specifically to the antibody binding to the inventive antigen, i.e. NECAB1 or a variant thereof. Said epitope may demonstrate strongest binding to antibody binding to the respective native antigen compared with the binding observed towards other antibodies. In one embodiment, such polypeptide comprises a stretch of 6, 7, 8, 9, 10, 11, 12, 20, 25, 30, 40, 50, 60, 70, 80, 90, 100, 113, 125 or more, preferably at least 9 but no more than 16, consecutive amino acids from the inventive antigen. The person skilled in the art is familiar with guidelines used to design peptides having sufficient immunogenicity, for example those described in Jackson, D. C., Fitzmaurice, C. J., Brown, L. E., Zeng, W. (1999), Preparation and properties of totally synthetic immunogenes, Vaccine Volume 18, Issues 3-4, September 1999, Pages 355-361; and Black, M., Trent, A., Tirrell, M. and Olive, C. (2010), Advances in the design and delivery of peptide subunit vaccines with a focus on Toll-like receptor agonists, Expert Rev Vaccines, 2010 February; 9(2): 157-173. Briefly, it is desirable that the peptide meets as many as possible of the following requirements: (a) it has a high degree of hydrophilicity, (b) it comprises one or more residues selected from the group comprising aspartate, proline, tyrosine and phenylalanine, (c) is has, for higher specificity, no or little homology with other known peptides or polypeptides, (d) it needs to be sufficiently soluble and (e) it comprises no glycosylation or phosphorylation sites unless required for specific reasons. Alternatively, bioinformatics approaches may be followed, for example those described by Moreau, V., Fleury, C., Piquer, D., Nguyen, C., Novali, N., Villard, S., Laune, D., Granier, C. and Molina, F. (2008), PEPOP: Computational design of immunogenic peptides, BMC Bioinformatics 2008, 9:71.

The inventive polypeptide, when used according to the present invention, may be provided in any kind of conformation. For example, the polypeptide may be an essentially unfolded, a partially or a fully folded polypeptide. In a preferred embodiment, the polypeptide is folded in the sense that the epitopes essential for the binding to the inventive antibody, or the protein or variant thereof in its entirety, adopt the fold adopted by the native protein in its natural environment. The person skilled in the art is familiar with methods suitable to determine whether or not a polypeptide is folded and if it is, which structure it has, for example limited proteolysis, NMR spectroscopy, CD spectroscopy or X-ray crystallography (see for example Banaszak L. J. (2008), Foundations of Structural Biology, Academics Press, or Teng Q. (2013), Structural Biology: Practical Applications, Springer), preferably CD spectroscopy is used.

The inventive polypeptide may be a fusion protein which comprises amino acid sequences other than those taken from the inventive antigen, in particular a C-terminal or N-terminal tag, preferably a N-terminal tag, which is, in a preferred embodiment, as used herein, an additional sequence motif or polypeptide having a function that has some biological or physical function and may, for example, be used to purify, immobilize, precipitate or identify the inventive polypeptide. In a more preferred embodiment, the tag is a sequence or domain capable of binding specifically to a ligand, for example a tag selected from the group comprising His tags, thioredoxin, maltose binding protein, glutathione-S-transferase, a fluorescence tag, for example from the group comprising green fluorescent protein.

In a preferred embodiment, the term "sensitivity" refers to the number of samples correctly determined as positive relative to the total number of samples examined. In a preferred embodiment, the term "specificity" refers to number of samples correctly determined as negative relative to the total number of samples examined.

The variant has biological activity. In a preferred embodiment, such biological activity is the ability to bind specifically to an antibody binding specifically to the corresponding antigen, such as NECAB1, as found in a patient suffering from an autoimmune disease associated with the presence of such antibody in a sample, preferably selected from the group comprising PNS, dementia, psychotic symptoms, acoustic hallucinations, seizures, encephalitis, such as limbic encephalitis, brainstem encephalitis or autoimmune encephalitis, peripheral polyneuropathy, neuronopathy, such as senso-motoric neuronopathy, Guillain-Barré syndrome (GBS) symptoms, encephalopathy, encephalomyelitis, cerebellitis, encephalomyelitis, myelitis, tetraparesis, cerebellar/brainstem syndrome, hearing loss, symmetric maculopathy, cerebellar syndrome, cerebellopontine lesion extending into spinal cord, personality changes, cognitive impairment and affect disorder, gait disorder, gait and stand ataxia, multiple cerebral ischemia, and cancers such as carcinoma, cerebral metastases, lung cancer, such as lung carcinoma, mamma carcinoma and melanoma, even more preferably selected from the group comprising PNS, personality changes, cognitive impairment and affect disorder, gait disorder, encephalitis, such as limbic encephalitis, brainstem encephalitis or autoimmune encephalitis, mamma carcinoma and lung cancer, such as lung carcinoma. In preferred embodiments, the dementia is a rapidly progressive dementia. In other preferred embodiments, the encephalitis is limbic encephalitis, brainstem encephalitis or autoimmune encephalitis. In further preferred embodiments, the carcinoma is a mamma carcinoma or a lung carcinoma such as SCLC or NSCLC. For example, whether or not a variant of the polypeptide has biological activity may be checked by determining whether or not it binds specifically to an antibody from a sample of such a patient comprising an antibody binding specifically to wild type antigen, preferably as determined by indirect immunofluorescence as described in the experimental section of this application. In a preferred embodiment, the person skilled in the art will, when designing variants consider that domains exposed to the cytoplasm or extracellular space are likely to contain epitopes of the antibody to be detected. In even more preferred embodiments, the variants may be a polypeptide comprising at least one sequence set forth in SEQ ID NO2, SEQ ID NO3, SEQ ID NO4, SEQ ID NO5, SEQ ID NO6, SEQ ID NO7, SEQ ID NO8, SEQ ID NO9, SEQ ID NO10, SEQ ID NO18, SEQ ID NO19 and/or SEQ ID NO20.

According to the present invention, a cell is provided which overexpresses a polypeptide comprising an antigen or at least one epitope thereof or a variant of the antigen or epitope thereof, wherein the antigen is NECAB1, preferably in combination with at least one additional cell which overexpresses another antigen from the group consisting of Hu, Yo, Ri, CV2, PNMA1, PNMA2, DNER/Tr, ARHGAP26, ITPR1 (EP14003703.7), ATP1A3, NBC1 (EP14003958.7), Neurochrondrin (EP15001186), CARPVIII, Zic4, SOX1 (US7314721), Ma, MAG, MP0, MBP, GAD65, amphiphysin, recoverin, GABA A receptor, GABA B receptor, glycine receptor, gephyrin, IgLON5, DPPX, aquaporin-4, MOG, NMDA receptor, AMPA receptors, GRM1, GRM5, LGI1, VGCC, mGluR1, CASPR2, ATP1A3, also referred to as alpha 3 subunit of human neuronal Na(+)/K(+) ATPase (EP14171561.5) and Flotillin1/2 (EP3101424) or a variant thereof comprised in a polypeptide.

In a preferred embodiment, the term "overexpressing", as used herein, means that the cell has been transfected with a nucleic acid that comprises a nucleic acid sequence encoding a polypeptide comprising the antigen of the invention or the other antigen or a variant thereof under the control of a promotor. Consequently, the transfected cell expresses more polypeptide recognized by the antibody binding specifically to be detected than the same type of cell normally would, probably at least 10, 20, 30, 50, 100, 200 or 500% more as judged by quantitative Western Blot. The promoter may be an inducible promoter, which allows for the induction of expression by addition of an inducer. The person skilled in the art is familiar with protocols and vectors for transiently overexpressing a polypeptide in a eukaryotic cell, for example the pTriEx system from Novagen and with protocols and vectors for stably transfecting a eukaryotic cell, for example the pcDNA^{™}4/TO vector system from Invitrogen.

In a preferred embodiment, a fixed mammalian cell may be used. In a preferred embodiment, the term "fixed" cell, as used herein, refers to a cell that has been treated with a reactive chemical compound to the effect that the cell is no longer metabolically active, but still presents its epitopes for immunostaining with antibodies and their subsequent detection, for example by fluorescence. More preferably, the reactive chemical compound is selected from the group comprising acetone, formalin, methanol and ethanol or mixtures thereof, preferably all of them. The person skilled in the art is familiar with protocols that may be used to prepare fixed cells.

According to the present invention, the cell may be on a carrier for microscopic immunofluorescence analysis. Such a carrier may be a glass slide. The cell on the glass slide may be covered with a mounting buffer. A mounting medium is a liquid which helps maintain a near physiological pH to maintain the molecular structure of any diagnostically relevant molecule and their epitopes, is compatible with the emission of a fluorescence signal and prevents a premature loss of fluorescence due to bleaching of the fluorophore. It may be selected from the group consisting of water, glycerol, natural oil or plastic or a mixture thereof, preferably water and glycerol. Various compositions are described in the state of the art, for example in "Mountants and Antifades", published by Wright Cell Imaging Facility, Toronto Western Research Institute University Health Network, (https://de.scribd.com/document/47879592/Mountants-Antifades), Krenek et al. (1989) J. Immunol. Meth 117, 91-97 and Nairn et al. (1969) Clin. Exp. Immunol. 4, 697-705.

A cover glass may be placed on top of the composition comprising the sample and the mounting medium. Slides with cover glasses (FB 112d-1005-1 or ZZ 3000-0112) are available from EUROIMMUN Medizinische Labordiagnostika, AG. However, any carrier compatible with microscopic analysis of the fluorescence pattern may be used. The carrier may comprise a mock-transfected cell, which has been transfected with the same vector as the cell overexpressing a polypeptide comprising an antigen or at least one epitope thereof or a variant of the antigen or epitope thereof, wherein the antigen is NECAB1 or a variant thereof, but without the nucleic acid encoding for the latter. Such mock-transfected cell may serve as a negative control. The carrier is configured for analysis using an immunofluorescence microscope.

In a preferred embodiment, the carrier may comprise a field comprising the cell according to the invention. In addition, the carrier may comprise additional fields. The fields are preferably surrounded by a hydrophobic surface. Each of these fields may comprise a cell overexpressing another antigen or a variant thereof. A field may comprise a section of primate cerebellum, rat cerebellum or rat hippocampus and thalamus. In a preferred embodiment, the cell is a eukaryotic cell overexpressing the polypeptide, such as a cell selected from the group comprising HEK, Hela, CHO and Jurkat cells and derivatives thereof. In a preferred embodiment, the cell is a recombinant cell overexpressing the polypeptide, which is preferably under the control of a heterologous strong promoter.

In another preferred embodiment, the carrier such as the diagnostically useful carrier is a bead. Various beads for numerous applications are commercially available, mainly based on carbohydrate, for example sepharose or agarose, or plastic. They may contain active or activatable chemical groups such as a carboxyl or tosyl or ester group, which can be utilized for the immobilization of a means for specifically capturing an antibody. Preferably, the beads are beads having an average diameter of from 0.1 µm to 10 µm, from 0.5 µm to 8 µm, from 0.75 µm to 7 µm or from 1 µm to 6 µm. Preferably, the bead is provided in the form of an aqueous suspension having a bead content of from 10 to 90%, preferably from 20 to 80%, preferably from 30 to 70%, more preferably from 40 to 60% (w/w). The person skilled in the art is familiar with such beads (Diamindis, E. P., Chriopoulus, T. K., Immunoassays, 1996, Academic Press), which are commercially available, for example Bio-Plex COOH beads MC10026-01 or 171-506011 from Bio-Rad.

In another preferred embodiment, the carrier is a microtiter plate comprising at least 8 wells that may be used for ELISA. At least one of the wells is coated with the means for specifically capturing an antibody, either directly or indirectly, preferably a polypeptide comprising SEQ ID NO1, SEQ ID NO2, SEQ ID NO3, SEQ ID NO4, SEQ ID NO5, SEQ ID NO6, SEQ ID NO7, SEQ ID NO8, SEQ ID NO9 and/or SEQ ID NO10 or a variant thereof. At least 3, preferably 4, more preferably 5 calibrators, at defined concentrations may be used to set up a calibration curve for semi-quantitative analysis. When the inventive method is carried out, the calibrators, which typically cover a range of concentrations covering the calibrating curve, may be processed and developed in parallel to the samples. A secondary antibody comprising a detectable label such as an enzymatically active label may be provided, for example a label having horse radish peroxidase activity or alkaline phosphatase activity or an enzyme capable of chemiluminescence.

In another preferred embodiment, the carrier is a microarray. In a preferred embodiment, the term "microarray", as used herein, refers to a chip spotted with a variety of spatially separate antigens, preferably at least 5, more preferably 10, 20, 30, 40, 50, 80 or 100. Preferably each antigen is a peptide comprising or consisting of 5 to 25, preferably 7 to 15 successive amino acids spanning a fragment of a mammalian protein. At least one antigen is a polypeptide comprising NECAB1 or an epitope thereof or a variant of NECAB1 or the epitope thereof. Preferably two or more antigens are a polypeptide comprising NECAB1 or an epitope thereof or a variant of NECAB1 or the epitope thereof, for example SEQ ID NO1, SEQ ID NO2, SEQ ID NO3, SEQ ID NO4, SEQ ID NO5, SEQ ID NO6, SEQ ID NO7, SEQ ID NO8, SEQ ID NO9, SEQ ID NO10, SEQ ID NO18, SEQ ID NO19 and/or SEQ ID NO20 or a variant thereof. A secondary antibody comprising a label, preferably a fluorescent label, and specifically binding to human immunoglobulin, preferably human IgG may be used for the detection. Preferably at least one additional antigen or a variant thereof is spotted.

In a preferred embodiment, the presence of an antibody binding to NECAB1 or a variant thereof in a sample from a subject is determined using ELISA. In another preferred embodiment, the presence of an antibody binding to NECAB1 or a variant thereof in a sample from a subject is determined using immunofluorescence, preferably indirect immunofluorescence.

According to the present invention, a means for detecting an antibody is used, which is a molecule binding specifically to the antibody and allowing detection, typically comprising a detectable label. In a preferred embodiment, the means for detecting an antibody is a secondary antibody or a polypeptide comprising an antigen or at least one epitope thereof or a variant of the antigen or epitope thereof, wherein the antigen is NECAB1 or a variant thereof. In a preferred embodiment, a label as used herein is a detectable label. In a preferred embodiment, a detectable label may be used to distinguish a population of molecules from others using biophysical detection methods. It is preferably selected from the group comprising a fluorescent, a radioactive, a chemiluminescent label, a heavy metal such as gold label, a nanoparticle, a bead or an enzymatically active label, preferably one catalyzing a colorimetric reaction. In a preferred embodiment, a fluorescent label is selected from the group comprising Alexa dyes, FITC, TRITC and green fluorescent protein (GFP). Iodine-125 may be used as radioactive label. In a preferred embodiment, an enzymatically active label is selected from the group comprising horseradish peroxidase, glucose oxidase, beta galactosidase, alkaline phosphatase and luciferase. In a preferred embodiment, a chemiluminescent label is selected from the group comprising luminol or a derivative, an acridinium ester and luciferase. The person skilled in the art is able to choose suitable labels and to attach them to proteins, nucleic acids and other molecules (Hassanzadeh L, Chen S, Veedu RN. Radiolabeling of Nucleic Acid Aptamers for Highly Sensitive Disease-Specific Molecular Imaging. Pharmaceuticals (Basel). 2018;11(4):106. Published 2018 Oct 15. doi:10.3390/ph11040106, Bioconjugate Techniques, 3rd Edition (2013) by Greg T. Hermanson, Obermaier C, Griebel A, Westermeier R. Principles of protein labeling techniques. Methods Mol Biol. 2015; 1295: 153-65), and a wide range of labeled molecules are commercially available. According to the present invention, a means for capturing an antibody such as an IgG class antibody is a molecule binding specifically to the antibody to be captured and/or to be immobilized and which may be capable of immobilizing it, either because it is immobilized itself or configured for immobilization, preferably *via* an affinity tag. Preferably, the means for capturing the antibody is a secondary antibody or a polypeptide comprising NECAB1 or an epitope thereof or a variant of NECAB1 or the epitope thereof.The means for detecting an antibody such as an immobilized antibody may be a ligand binding specifically to mammalian immunoglobulin, preferably human immunoglobulin, more preferably human IgG, and/or mammalian, preferably human NECAB1 or a variant thereof and/or may be selected from the group comprising a secondary antibody, a polypeptide comprising NECAB1 or at least one epitope thereof or a variant thereof, a ligand binding specifically, Protein G or a variant thereof, Protein A or variant thereof, or an aptamer or an antibody binding specifically to the antibody. Preferably, the means for detecting the antibody is a secondary antibody or a polypeptide comprising NECAB1 or an epitope thereof or a variant of NECAB1 or the epitope thereof. For example, the means for capturing an antibody specifically binding to NECAB1 may be a secondary antibody and the means for detecting the antibody may be a polypeptide comprising NECAB1 or an epitope thereof or a variant of NECAB1 or the epitope thereof, which is optionally labeled, preferably with a detectable label. As another example, the means for capturing an antibody specifically binding to NECAB1 may be a polypeptide comprising NECAB1 or an epitope thereof or a variant of NECAB1 or the epitope thereof and the means for detecting the antibody may be a secondary antibody, which is optionally labeled, preferably with a detectable label.

In accordance with the present invention, the term "secondary antibody" in its broadest sense is to be understood to refer to any kind of "binding moiety", preferably binding protein, capable of specific binding to immunoglobulins, preferably mammalian, more preferably human immunoglobulins, more preferably to an IgA, IgG and/or IgM class antibody or a fragment thereof such as a constant domain of a particular Ig class of a selected species, preferably human species. In one embodiment, the secondary antibody binds specifically to one or more sequences, or binds specifically to an antibody comprising one or more sequences selected from the group comprising SEQ ID NO24, SEQ ID NO25, SEQ ID NO26, SEQ ID NO27, SEQ ID NO28, SEQ ID NO29, SEQ ID NO30, SEQ ID NO31, SEQ ID NO32, SEQ ID NO33, SEQ ID NO34, SEQ ID NO35, SEQ ID NO36, SEQ ID NO37 and SEQ ID NO38, preferably to one or more sequences selected from the group comprising SEQ ID NO28, SEQ ID NO29, SEQ ID NO30 and SEQ ID NO31. Non-limiting examples of binding moieties include antibodies, for example antibodies immunologically or genetically derived from any species, for example human, chicken, camel, llama, lamprey, shark, goat, rodent, cow, dog, rabbit, etc., antibody fragments, domains or parts thereof, for example Fab, Fab', F(ab')₂, scFab, Fv, scFv, VH, VHH, VL, VLRs, and the like, diabodies, monoclonal antibodies (mAbs), polyclonal antibodies (pAbs), mAbdAbs, phage display-derived binders, affibodies, heteroconjugate antibodies, bispecific antibodies, evibodies, lipocalins, anticalins, affibodies, avimers, maxibodies, heat shock proteins such as GroEL and GroES, trans-bodies, DARPins, aptamers, C-type lectin domains such as tetranectins; human γ-crystallin and human ubiquitin-derived binders such as affilins, PDZ domain-derived binders; scorpion toxin and/or Kunitz-type domain binders, fibronectin-derived binders such as adnectins, receptors, ligands, lectins, streptavidin, biotin, including derivatives and/or combinations thereof such as bi-/multi-specific formats formed from two or more of these binding molecules. Various antibody-derived and alternative (i.e. non-antibody) binding protein scaffolds including methods of generation thereof are known in the art (e.g. reviewed in Chiu ML et al., Antibodies (Basel), (2019) 8(4):55; Simeon R. & Chen Z., Protein Cell. (2018) 9(1):3-14; and Chapter 7 - Non-Antibody Scaffolds from Handbook of Therapeutic Antibodies (2007) edited by Stefan Dübel, US 7,166,697, Rothe C & Skerra A., BioDrugs. (2018) 32(3):233-243; Gebauer M & Skerra A, Curr Opin Biotechnol. (2019) 60:230-241; Feldwisch, J & Tolmachev, V. (2012) Methods Mol. Biol. 899:103-126; Wikman M et al., Protein Eng Des Sel. (2004) 17(5):455-62; Silverman J et al. (2005), Nat Biotechnol 23:1556-1561; Plückthun A., Annu Rev Pharmacol Toxicol. (2015) 55:489-511; Hosse RJ et al. (2006) Protein Sci 15:14-27; Hackel BJ, et al. (2008) J Mol Biol 381:1238-1252. In a preferred embodiment, a secondary antibody is an antibody binding to all antibodies from an antibody or immunoglobulin class, preferably a human antibody class, preferably IgA and/or IgG and/or IgM antibodies, preferably IgG. Secondary antibodies may recognize the constant domain of said class or one or more epitopes across the sequence or 3D structure shared by antibodies of the Ig class of interest. Secondary antibodies are typically from a mammal other than a human or from a bird, preferably from chicken, rabbit, mouse, rat, horse, pig, donkey, goat, cow, camel, llama, or non-human primate. A secondary antibody may be a monoclonal, preferably recombinant antibody or may be a polyclonal antibody. A wide range of them is commercially available. In preferred embodiments, the secondary antibody specifically binds to human IgG, more preferably to the constant region of human IgG.

In a preferred embodiment, a ligand to an affinity tag, as used herein, is an artificial entity binding specifically to an affinity tag, typically a chemically synthesized modification or a recombinant protein or peptide attached to a molecule of interest. The ligand to an affinity tag depends on the type of affinity tag chosen and may be selected from the group consisting of His, immobilized nickel, glutathione, chitin, 18A, ACP, Aldehyd, Avi, BCCP, Calmodulin, Chitin binding protein, E-Tag, ELK16, FLAG, flash, poly glutamate, poly aspartate, GST, GFP, HA, Isope, maltose binding protein, myc, nus, NE, ProtA, ProtC, Tho1d4, S-Tag, SnoopTag, SpyTag, SofTag, Streptavidin, Strep-tag II, T7 Epitope Tag, TAP, TC, Thioredoxin, Ty, V5, VSV, biotin, Xpress Tag and a recombinant antibody binding to the ligand to an affinity tag.

According to the present invention, an antibody binding specifically to NECAB1 is provided, preferably in a solution comprising one or more, more preferably all from the group comprising an artificial buffer, a preservative and an artificial anticoagulant. An artificial buffer is a buffer which is synthetic and/or may not occur in the body of the patient or at least at concentrations well below the concentration used. The buffer may be selected from the group consisting of Tris, phosphate, Tricine, acetate, MOPS, MES, carbonate, citrate and HEPES. In a preferred embodiment, the term "preservative" as used herein, refers to a substance inhibiting microbial growth and/or chemical degradation in a liquid solution and may preferably be selected from the group consisting of azide, lactic acid, nitrate, nitrite, antibiotics, a protease inhibitor and ethanol.

Various methods or uses according to the invention can be conducted with a sample from a subject as described herein. These methods or uses can also be characterized as "*in vitro*" methods or "*in vitro*" uses.

In a preferred embodiment, the term "chemical solution reactive with a detectable label" refers to a compound in a liquid which, upon exposure to the detectable label, emits a detectable signal. The solution may comprise a chromogenic substrate of an enzymatically active label. For example, 3,3', 5,5' tetramethylbenzidine/H₂O₂ may be used if the label is a peroxidase. The solution may comprise a small inorganic or organic compound capable of reacting with a chemiluminescent label. In the case of an acridinium ester, a mixture of H₂O₂ and sodium hydroxide is frequently used as the chemical solution. Various other chemical solutions and detectable labels are known in the art (Weeks, I., Beheshti, I., McCapra, F., Campbell, A. K., Woodhead, J. S. (1983) Acridinium esters as high specific activity labels in immunoassay. Clin Chem 29: 1474-1479), Thermo Scientific Pierce Antibody Production and Purification Technical Handbook, Version 2, www.thermoscientific.com).

In a preferred embodiment, the term "diagnosis", as used herein, is to be used in its broadest possible sense and may refer to any kind of procedure aiming to obtain information instrumental in the assessment whether a patient, known or an anonymous subject from a cohort, suffers or is likely or more likely than the average or a comparative subject, the latter preferably having similar symptoms, to suffer from certain a disease or disorder in the past, at the time of the diagnosis or in the future, to find out how the disease is progressing or is likely to progress in the future or to evaluate the responsiveness of a patient or patients in general with regard to a certain treatment, for example the administration of immunosuppressive drugs, or to find out whether a sample is from such a patient. Such information may be used for a clinical diagnosis but may also be obtained by an experimental and/or research laboratory for the purpose of general research, for example to determine the proportion of subjects suffering from the disease in a patient cohort or in a population. In other words, the term "diagnosis" comprises not only diagnosing, but also prognosticating and/or monitoring the course of a disease or disorder, including monitoring the response of one or more patients to the administration of a drug or candidate drug, for example to determine its efficacy. While the result may be assigned to a specific patient for clinical diagnostic applications and may be communicated to a medical doctor or institution treating said patient, this is not necessarily the case for other applications, for example in diagnostics for research purposes, where it may be sufficient to assign the results to a sample from an anonymized patient. Thus, in some embodiments, the person to be diagnosed, *i.e*., the "subject" or "patient", is an anonymous blood donor whose blood may be donated or used to obtain therapeutically or diagnostically useful antibodies. The term "diagnosis" also refers to negative diagnosis, *i.e*., the case where no antibodies specifically binding to NECAB1 or a variant thereof are found in a sample from a patient. In these embodiments, the absence of antibodies specifically binding to NECAB1 or a variant thereof indicates that the patient may suffer from a disease other than a disease associated with the presence of said antibodies, as described herein. Thus, in one embodiment, "diagnosis" also includes the case where the absence of an antibody specifically binding to NECAB1 or a variant thereof helps excluding certain diseases, such as the diseases associated with the presence of such antibody, as described herein, which may lead to or help in or support the indirect diagnosis of another disease. In another preferred embodiment, the detection of an antibody binding specifically to NECAB1 or a variant thereof is considered to imply a definitive diagnosis of a neurological autoimmune disease because of the presence of the antibody.

Thus, in a preferred embodiment, the term "diagnosis", "diagnosing" or "diagnostic" encompasses diagnosis, prognosis, theragnosis and monitoring in an autoimmune disease, preferably a neurological autoimmune disease, such as those described herein, and/or a cancer, such as those described herein. As used herein, the term "theragnosis" refers to the identification, for example by diagnostic methods, of patients who might benefit from a particular therapy and, optionally, the subsequent treatment of said patients.

In a preferred embodiment, the methods and products according to the present invention may be used for interaction studies, including determining whether a drug candidate or other compound may interfere with the binding of an antibody binding specifically to NECAB1 or a variant thereof or may affect any downstream process or the strength of its binding to its target. In preferred embodiment, they may be used for monitoring the immune response, more preferably the emergence and/or titer of antibodies to the antigen of the invention, following the administration of an immunogenic composition comprising a polypeptide comprising NECAB1 or a variant thereof or an immunogenic variant thereof, for example to a mammal, which may be a mammal other than a human such as a laboratory animal.

In a preferred embodiment, the methods and products may be used for providing reagents such as an antibody to NECAB1 or a variant thereof which may serve as a positive control or a calibrator for a diagnostic test or for developing and/or validating a diagnostic test. In a preferred embodiment, the term "validating", as used herein, refers to a procedure for establishing an assay in a specific environment based on a previously known principle and confirming that it yields useful results. For example, while this application discloses the usefulness of an antibody to an antigen of the invention as a marker for a diagnosis, a clinical or research laboratory may need to confirm the diagnostic value of the results before routinely using the test for their patients or a new group of patients, for example a group of animals, which may not have been known previously to suffer from a disease or condition.

In another preferred embodiment, the methods and products according to the present invention may be used for determining the concentration of an antibody binding specifically to NECAB1 or a variant thereof. In a more preferred embodiment, said antibody is an autoantibody from a patient suffering from a neurological autoimmune disease. In another preferred embodiment, said antibody is a recombinant antibody which binds to NECAB1 or a variant thereof, but is recognized by a secondary antibody binding specifically to human immunoglobulin, preferably human IgG class antibodies, preferably IgG1, IgG2, IgG3 and IgG4 isotypes. In a more preferred embodiment, such a concentration needs to be determined for the purposes of research, for the preparation or for monitoring the quality of reagents, animal models or devices that may or may not be used for the diagnosis of a neurological autoimmune disease.

In many cases the mere detection of the antibody, in other words determining whether or not detectable levels of the antibody are present in the sample, is sufficient for the diagnosis. In a more preferred embodiment, this may involve determining whether the concentration is at least 10%, preferably 20%, 50%, 100%, 200%, 500%, 1.000%, 2.000%, 2.500%, 5.000%, 1.0000%, 2.0000%, 5.0000%, 100.000%, 1.000.000% or 10.000.000% times higher than the concentration of the antibody of interest found in the average healthy subject. If the antibody can be detected, this will be information instrumental for the clinician's diagnosis. It may indicate an increased likelihood that the patient suffers from a disease.

The person skilled in the art will appreciate that a clinician does usually not arrive at the conclusion whether or not the patient suffers or is likely to suffer from a disease, condition or disorders solely on the basis of a single diagnostic parameter, but needs to take into account other aspects, for example the presence of other antibodies, markers, blood parameters, clinical assessment of the patient's symptoms or the results of medical imaging or other non-invasive methods such as polysomnography, to arrive at a conclusive diagnosis. See Baenkler H. W. (2012), General aspects of autoimmune diagnostics, in Renz, H., Autoimmune diagnostics, 2012, de Gruyter, page 3. The value of a diagnostic agent or method may also reside the possibility to rule out one disease, thus allowing for the indirect diagnosis of another. In a preferred embodiment, the meaning of any symptoms or diseases referred to throughout this application is in line with the person skilled in the art's understanding as of the filing date or, preferably, earliest priority date of this application as evidenced by text books and scientific publications. In a preferred embodiment, the methods or uses or products are not used, taken alone, to arrive at a definite, final diagnosis.

In a preferred embodiment, any information or data demonstrating the presence of absence of the antibody may be communicated to the patient or a medical doctor treating the patient orally, preferably by telephone, in a written form, preferably fax or letter, or in an electronic form *via* fax or *via* the internet, for example as an email or text message.

The inventive teachings may also be used in a method for preventing or treating a disease, preferably after a diagnosis according to the present invention, comprising the steps a) reducing the concentration of antibodies binding to NECAB1 or a variant thereof, in the subject's blood and/or b) administering one or more immunosuppressive pharmaceutical substances, preferably selected from the group consisting of rituximab, prednisone, methylprednisolone, cyclophosphamide, mycophenolatemofetil, intravenous immunoglobulin, tacrolimus, cyclosporine, methotrexate and azathioprine.

According to the present invention, the presence of an antibody may be determined in a qualitative or a quantitative manner. In a preferred embodiment, the term "detecting in a quantitative manner", as used herein, means that not only the presence of an antibody is detected, but that a result is obtained that includes information regarding the absolute or relative amount of the antibody in the sample. In a more preferred embodiment, a value representing an absolute concentration is obtained. In another more preferred embodiment, a value representing a relative concentration or change of concentration is obtained. In another preferred embodiment, also referred to as "semi-quantitative" approach, the concentration of the antibody is placed in one of several groups, most preferably a concentration window meaning that it is virtually absent, a concentration window meaning that a borderline result is obtained and a concentration window meaning that the antibody is present. A further distinction into categories such as "weak positive" or "strong positive" signal is possible.

In a preferred embodiment, the term "autoantibody", as used herein, refers to an antibody binding specifically to an endogenous molecule of the animal, preferably mammal, more preferably human, which produces said autoantibody, wherein the level of such antibody is more preferably elevated compared to the average healthy subject. Thus, an autoantibody is an endogenous molecule produced within a subject's body. The subject is preferably a patient suspected of or actually suffering from a disease, preferably a mammalian, more preferably human patient. Such an autoantibody has a constant region, as have other antibodies of the same class from the same organism. Particularly preferably, the autoantibody is a mammalian autoantibody, even more preferably a human autoantibody, even more preferably a human autoantibody of class IgG, IgM or IgA, preferably IgG. The variable domain thereof is capable of binding specifically against the antigen described herein. In one embodiment, the constant domain binds specifically to molecules recognizing the constant domain of human immunoglobulin, preferably IgG class antibodies such as secondary antibodies. Thus, the autoantibody may have the sequence of an antibody's constant regions from the animal, preferably human, making it, but the variable region is able to bind specifically to the endogenous molecule of the animal, more specifically to NECAB1 or a variant thereof, even more preferably to SEQ ID NO1, SEQ ID NO2, SEQ ID NO3, SEQ ID NO4, SEQ ID NO5, SEQ ID NO6, SEQ ID NO7, SEQ ID NO8, SEQ ID NO9, SEQ ID NO10, SEQ ID NO18, SEQ ID NO19 and/or SEQ ID NO20. In a preferred embodiment, the autoantibody is isolated and/or purified from a sample, preferably tissue, serum, plasma, blood or CSF from the animal, preferably human. The autoantibody can be isolated as a mixture of polyclonal, native antibodies from the animal or patient. It is not a synthetic, monoclonal or recombinant antibody. A recombinant antibody binding specifically to NECAB1 or a variant thereof may be generated using standard methods. The autoantibody or the recombinant antibody may be useful as a positive control and for detection assays, for example sandwich assays or competitive assays.

The term "isolated" as used herein means that the molecule referred to as being isolated is free or essentially free of at least one component as it is found in nature. For example, the molecule, such as an antibody, may be free or essentially free from some or all components as it is found in its natural environment. Such components may comprise, for example in the case of an antibody, erythrocytes, leucocytes, thrombocytes, plasma, proteins, nucleic acids, salts, lipids, and nutrients.

The method according to the present invention is preferably an *in vitro* method.

According to the present invention, a polypeptide, preferably the polypeptide comprising an antigen or at least one epitope thereof or a variant of the antigen or epitope thereof, wherein the antigen is NECAB1 or a variant thereof, may be a recombinant protein. In a preferred embodiment, the term "recombinant", as used herein, refers to a polypeptide produced using genetic engineering approaches at any stage of the production process, for example by fusing a nucleic acid encoding the polypeptide to a strong promoter for overexpression in cells or tissues or by engineering the sequence of the polypeptide itself. The person skilled in the art is familiar with methods for engineering nucleic acids and polypeptides encoded (for example, described in Sambrook, J., Fritsch, E. F. and Maniatis, T. (1989), Molecular Cloning, CSH or in Brown T. A. (1986), Gene Cloning - an introduction, Chapman & Hall) and for producing and purifying native or recombinant polypeptides (for example Handbooks "Strategies for Protein Purification", "Antibody Purification", published by GE Healthcare Life Sciences, and in Burgess, R. R., Deutscher, M. P. (2009): Guide to Protein Purification). In another preferred embodiment, a polypeptide provided or used according to the present invention such as a polypeptide comprising an antigen or at least one epitope thereof or a variant of the antigen or epitope thereof, wherein the antigen is NECAB1, or an antibody is an isolated polypeptide, wherein the term "isolated" means that the polypeptide has been enriched compared to its state upon production using a biotechnological or synthetic approach and is preferably pure, *i.e.* at least 60, 70, 80, 90, 95 or 99 percent of the polypeptide in the respective liquid consists of said polypeptide as judged by SDS polyacrylamide gel electrophoresis followed by Coomassie blue staining and visual inspection. Preferably any polypeptide on a carrier used as a means to capture an antibody is pure.

Patients having antibodies to NECAB1 or a variant thereof may suffer from a variety of cancers comprising mamma carcinoma, lung carcinoma such as NSCLC, cerebral metastases and melanoma. In a preferred embodiment, the term "cancer", as used herein, refers to a group of diseases involving abnormal cell growth with the potential to invade or spread to other parts of the body.

In a preferred embodiment, the term "mamma carcinoma" or "breast cancer", as interchangeably used herein, is defined as cancer which originates in the breast, whereas the cells have malignant form and structure, the ability for uncontrolled growth and the potential or ability to invade or metastasize. In a preferred embodiment, the breast cancer spreads to other organs, such as lymph nodes. In further preferred embodiments, the breast cancer is invasive and may be metastatic.

In a preferred embodiment, the term "non-small cell lung carcinoma (NSCLC)", as used herein, includes epidermoid carcinoma cells, adenocarcinoma cells, and large cell undifferentiated carcinoma cells. The most common symptoms associated with NSCLC are coughing (including coughing up blood), weight loss, shortness of breath, and chest pains.

In a preferred embodiment, the term "cerebral metastases", as used herein, refers to tumors, which originate from outside the Central Nervous System (CNS, such as brain and spinal cord) and spread to it via the blood stream or directly invade from neighboring tissues. Metastatic tumors are the commonest tumors that affect the brain and spinal cord.

In a preferred embodiment, the term "melanoma", as used herein, refers to a malignant tumor of melanocytes. Melanocytes are cells that produce the dark pigment, melanin, which is responsible for the color of skin. They predominantly occur in skin but are also found in other parts of the body, including the bowel and the eye. Melanoma can occur in any part of the body that contains melanocytes.

Additional background and definitions related to cancers and their diagnoses or differential diagnoses including the detection of antibodies may be taken from neurology textbooks available at the earliest priority date or filing date of this publication such as Kaye, Textbook of Medical Oncology, 3rd edition, Taylor & Francis; Shoenfeld, Meroni and Gershwin, Autoantibodies 3rd edition, Elsevier, in particular Part 11 including Chapter 76; and Darnell and Posner, Paraneoplastic Syndromes, Oxford University Press, 2011.

Patients having antibodies to NECAB1 or a variant thereof suffer from a variety of autoimmune diseases, such as neurological autoimmune diseases, comprising PNS, dementia, psychotic symptoms, acoustic hallucinations, seizures, encephalitis, encephalopathy, encephalomyelitis, myelitis, cerebellitis, neuropathy, dementia, peripheral polyneuropathy, Guillain-Barré syndrome (GBS) symptoms, tetraparesis, cerebellar/brainstem syndrome, hearing loss, symmetric maculopathy, cerebellopontine lesion extending into spinal cord, personality changes, cognitive impairment and affect disorder, gait and stand ataxia, multiple cerebral ischemia, carcinoma, cerebral metastases and melanoma, preferably rapidly progressive dementia, limbic encephalitis, brainstem encephalitis, autoimmune encephalitis, mamma carcinoma and lung cancer, such as lung carcinoma (such as NSCLC).

In a preferred embodiment, the term "ataxia", as used herein, refers to lack of voluntary coordination of muscle movements that can include gait abnormality, speech changes, and abnormalities in eye movements. Ataxia is a clinical manifestation indicating dysfunction of the parts of the nervous system that coordinate movement, such as the cerebellum.

In a preferred embodiment, the term "polyneuropathy", as used herein, refers to a disease affecting peripheral nerves (peripheral neuropathy) in roughly the same areas on both sides of the body, featuring weakness, numbness, and burning pain.

In a preferred embodiment, the term "encephalitis", as used herein, refers to an inflammation of the brain, with symptoms including reduced or alternation in consciousness, personality changes, psychotic delusions, rigidity, headache, fever, confusion, a stiff neck, and vomiting. Complications may include seizures, hallucinations, trouble speaking, memory problems, and problems with hearing. The disease may be the result of an infection or may be an autoimmune disease, hence the detection of an antibody according to the invention may be used to distinguish these two types of encephalitis.

In a preferred embodiment, the term "dementia", as used herein, refers broadly to any disorder, disease, or syndrome characterized by an abnormal high and progressive loss of functional capacity of the brain. While symptoms of dementia can vary greatly, hallmarks of dementia include impairment of several core mental functions, including memory, communication and language, ability to focus and pay attention, reasoning and judgment, and visual perception. People with dementia may have problems with short-term memory, keeping track of a purse or wallet, paying bills, planning and preparing meals, remembering appointments or traveling out of the neighborhood. Many dementias are progressive, meaning symptoms start out slowly and gradually get worse. Dementia may be determined using standard clinical procedures, with the degree of dementia being defined by the score in the Mini Mental State Examine (MMSE), as detailed in Folstein M.F., Folstein S.E. and McHugh P.R., J Psychiatry Res., 12:189-198 (1975). Examples of dementia or a dementia related neurological disorder include, but are not limited to, Alzheimer's Disease (AD), progressive supranuclear palsy (PSP), Huntington's Disease (HD), dementia of mixed type, Parkinson's Disease, diffuse Lewy Body dementia, vascular dementia, frontotemporal dementia, semantic dementia and dementia with Lewy bodies. In more preferred embodiments, the dementia is a rapidly progressive dementia.

In a preferred embodiment, the term "ischemia", as used herein, refers to a condition resulting from a decrease or lack of blood flow and oxygen to a part of the body such as the brain, heart, or other tissue. Ischemic injury refers generally to the damage to a tissue that is distal or otherwise effected by the loss of blood flow and oxygen. Ischemic injury is often a result of the lack of oxygen and fluids, but also includes inflammatory cascades. For example, ischemia and ischemic injury can occur as a result of cardiac, pulmonary or brain injury, organ transplantation or surgical procedure, or a disease or disorder (e.g., Sickle Cell Anemia or Sickle Cell Disease). In more preferred embodiments, the ischemia is multiple cerebral ischemia.

In a preferred embodiment, the term "Paraneoplastic Neurological Syndrome" (PNS), as used herein, refers to neurological syndromes associated with the presence of a cancer associated with tumors which presents an antigen which is normally exclusive to the nervous system. As a result, an antibody binding specifically to the neurological antigen is produced which may then damage the nervous system. Manifestations of PNS include, but are not limited to encephalitis, typically associated with seizures, psychiatric manifestations such as hallucinations, anxiety and depression, cerebellar symptoms, such as ataxia, nystagmus and dysarthria, opsoclonus-myoclonus and sensory neuronopathy; and Lambert-Eaton myasthenic syndrome. It should be mentioned that PNS-associated tumors are often small, grow slowly and may not yet be detectable when neurological syndromes surface. PNS may be associated with one or more antibodies, which may bind specifically to an antigen from the group comprising NMDAR, Lgl1, AMPA1, AMPA2, CASPR2, GABA B, GABA A, DPPX, IGLON5, Hu, Yo, CRMP5, Ri, Ma2, Amphiphysin, Recoverin, RGS8, DAGLA, NSF, STX1B, DNM1 and VAMP2, Hu, Ri, Ma, Anna-3, Zic-4, SOX1, Yo, PCA2, Tr and glutamic acid decarboxylase.

Additional background and definitions related to neurological syndromes and symptoms and their diagnoses or differential diagnoses including the detection of antibodies may be taken from neurology textbooks available at the earliest priority date or filing date of this publication such as Simon, Greenberg, Aminoff, Clinical Neurology, 7th edition, 2009, McGraw; Shoenfeld, Meroni and Gershwin, Autoantibodies 3rd edition, Elsevier, in particular Part 11 including Chapter 76; and Darnell and Posner, Paraneoplastic Syndromes, Oxford University Press, 2011.

The methods described herein for determining the presence of an antibody binding to NECAB1 or a variant thereof in a sample can be combined with methods for determining the presence of other antibodies, the presence of which is associated with the occurrence of the diseases described herein or of similar diseases. For example, one or more polypeptides selected from NECAB1 or a variant thereof, as described herein, can be used in conjunction with one or more polypeptides or variants thereof binding to other antibodies, the presence of which is associated with the occurrence of the diseases described herein or of similar diseases. For example, a device comprising a polypeptide comprising NECAB1 or an epitope thereof or a variant of NECAB1 or the epitope thereof, such as the device or carrier of the present invention, may comprise further polypeptides or variants thereof binding to other antibodies, the presence of which is associated with the occurrence of the diseases described herein or of similar diseases. Such device may be used to analyze a sample from a subject such as a patient for the presence of any antibody, the presence of which is associated with the occurrence of the diseases described herein or of similar diseases. In the case that a method using such a device or using several devices, wherein each device comprises at least one polypeptide or variant thereof binding to an antibody binding to NECAB1, or to another antibody the presence of which is associated with the occurrence of the diseases described herein or of similar diseases, determining the presence of any antibody binding to any of the assayed polypeptides or variants thereof may be sufficient for a skilled person to conclude on the presence of a certain disease or to limit the suspected disease to certain diseases or to aid in the diagnosis of a disease, as described herein, or to gain information about the risk of a subject to suffer in the present, past or future from or to develop a disease as described herein. Thus, under certain circumstances, it may be sufficient to determine the presence of either an antibody binding to NECAB1 or of any other antibody, the presence of which is associated with the occurrence of the diseases described herein or of similar diseases, without getting information or reaching a conclusion which specific antibody is present in a given sample, as long as the presence of said antibodies is associated with the occurrence of the same or similar disease(s). Consequently, determining the presence of an antibody binding to NECAB1 may comprise determining the presence of any antibody the presence of which is associated with the occurrence of the diseases described herein as being associated with NECAB1 or of similar diseases.

A person skilled in the art will understand that under certain circumstances, it may be instrumental to first analyze the clinical symptoms of a patient having or suspected of having a disease which is associated with the presence of an antibody binding to NECAB1. Depending on the symptoms, grade of impairment and other factors known in the art, a clinician or another medical or scientific personal may then determine whether the antibody titer of the subject to be analyzed is increased compared to a healthy subject or compared to the titer of the subject to be analyzed prior to the onset of symptoms or compared to the average. Subsequently or independently, one or more methods as described herein for determining the presence of an antibody binding to NECAB1 may be conducted to determine the presence of such antibody and/or to determine the presence of any antibody, the presence of which is associated with a disease as described herein. Thus, determining the presence of an antibody binding to NECAB1 may be used as a preinvestigation or as one investigation amongst others allowing a clinician to reach a conclusion whether or not a subject suffers or is likely to suffer in the present, past or future from a disease. Determining the presence of an antibody binding to NECAB1 may also be used to distinguish between drug or alcohol use or abuse or the presence of an infectious disease and the presence of an autoimmune disease, as described herein.

In certain embodiments, it may be instrumental to determine the Ig class of an antibody binding to NECAB1. Thus, in one embodiment, determining the presence of an antibody binding to NECAB1 comprises determining the Ig class of said antibody. In other embodiments, determining the presence of an antibody binding to NECAB1 does not comprise determining the Ig class of said antibody. Likewise, determining the presence of an antibody binding to NECAB1 of a certain Ig class comprises determining the presence of antibodies binding to NECAB1 of other Ig classes. Determining the presence of an antibody binding to NECAB1 of a certain Ig class may also comprise determining the absence of said antibody.

As part of a diagnosis relating to the neurological syndromes associated with NECAB1 associated antibodies, the clinician will initially consider a range of tests and risk factors which may point them either to autoimmune disease or infectious disease for many of the conditions associated with the presence of an antibody binding specifically to NECAB1 (Lancaster, J Clin Neurol. 2016 Jan;12(1) https://doi.org/10.3988/jcn.2016.12.1.1, Lee and Lee, The Laboratory Diagnosis of Autoimmune Encephalitis, Journal of Epilepsy Research 6 (2), 45), preferably encephalitis. Detection of an antibody binding specifically to NECAB1 will then confirm an autoimmune background, while the absence of an antibody will tempt the clinician to consider autoimmune disease associated with other antibodies. However, typically the presence or absence of a range of antibodies will then be detected, and a negative result will be a pointer to infectious diseases.

According to the present invention, a kit is provided, comprising the cell or the carrier, or the polypeptide and/or the means for capturing and/or the means for detecting an antibody specifically binding to NECAB1, and further comprising one or more, preferably all reagents from the group consisting of a secondary antibody, preferably labeled with a detectable label, a washing solution, a positive control, a negative control, a detergent, a cover glass, a mounting medium and a physiological salt solution, preferably PBS, or salt required to prepare it. In a preferred embodiment, the positive control is a diluted sample, preferably serum or CSF, from a patient suffering from a neurological autoimmune disease or a monoclonal antibody binding specifically to NECAB1. In a preferred embodiment, the kit comprises the means for capturing and/or the means for detecting an antibody specifically binding to NECAB1. The negative control may be a diluted sample from a healthy subject, for example a blood donor. The kit may comprise instructions how to carry out the assay. Preferably, the secondary antibody is a secondary antibody binding specifically to IgG class antibodies, preferably human IgG class antibodies, more preferably to the constant region of a human IgG class antibody. In other preferred embodiments, said secondary antibody may be labeled (as described above).

In a preferred embodiment, the present invention provides a use of the cell, the polypeptide, the carrier for the manufacture of a kit or a composition for the diagnosis of a disease, preferably associated with the presence of a mammalian antibody, preferably autoantibody, binding specifically to NECAB1.

In a preferred embodiment, any method or use according to the present invention may be intended for a non-diagnostic use, i.e. determining the presence of an antibody binding specifically to NECAB1, for a use other than diagnosing a patient. For example, the method or use may be for testing *in vitro* the efficiency of a medical device designed to remove an antibody from a patient's blood, wherein the testing is performed on a liquid other than patient's blood. After the use of the medical device with a patient, its capacity to remove antibody may be checked by running a solution comprising antibody binding specifically to NECAB1 through the device, followed by use of the method according to the present invention to confirm that less or no antibody is in the solution that has been passed through the device, i.e. showing that the device has still the capacity to remove antibody from the solution.

According to the present invention, the method may be used for testing the efficacy of a drug candidate which may be used to treat patients suffering from or likely to suffer from a neurological autoimmune disease. Such a drug candidate may be any molecule capable of interfering with the interaction between NECAB1 and the corresponding antibody.

In another preferred embodiment, the method may be for confirming the reliability of a diagnostic assay and may involve detecting an antibody binding specifically to NECAB1 in a solution, which is not a sample from a patient who requires a diagnosis, but is known to comprise an antibody binding specifically to NECAB1, preferably at a known concentration. For example, it may be a recombinant antibody or a sample diluted in a dilution buffer such as PBS from an anonymous patient whose identity cannot be traced back. Alternatively, the solution may be a negative control not comprising the antibody binding specifically to check the background. Such method may be run in parallel with, after or before a diagnostic method. In a preferred embodiment, any method or use according to the present invention may be intended for generating an antibody profile, preferably for detecting a disease in a mammal, preferably a human.

In a preferred embodiment, any method or use according to the present invention may be for identifying a subject at risk of suffering from or developing a disease and/or a tumor.

In a preferred embodiment, the method may be for detecting an antibody binding specifically to NECAB1 in a solution which is not a sample from a mammal to be diagnosed or for the purpose of providing a diagnosis.

In a preferred embodiment, the problem underlying the present invention is solved by a method comprising the step contacting a device comprising a solid phase on which a polypeptide comprising an antigen or at least one epitope thereof or a variant of the antigen or epitope thereof, wherein the antigen is NECAB1, is immobilized with a buffered solution comprising an antibody binding specifically to the antigen, which solution is preferably not a sample from a patient in need of a diagnosis, wherein preferably
a) the concentration of the antibody in the solution is known, and/or
b) the antibody is a recombinant antibody and/or
c) the medical or diagnostic device is contacted with two or more solutions comprising the antibody, wherein the two or more solutions have a different concentration of the antibody, and/or
d) the antibody is recognized by secondary antibodies binding specifically to IgG antibodies,
followed by detection of a signal which indicates whether or not the antibody has bound to the polypeptide, optionally a signal relating to the concentration of the antibody in the solution or solutions.

In a preferred embodiment, the present invention provides an apparatus for analyzing a sample from a patient to detect an antibody binding specifically to NECAB1, indicating an increased likelihood of a neurological autoimmune disease or of developing it, comprising:
a. a carrier, which contains a means for capturing the antibody from the sample when the sample is contacted with the carrier, preferably wherein the means is the cell or the polypeptide and the carrier is the carrier according to the present invention,
b. a means for detecting the antibody capable of binding to the antibody captured by the carrier when the detectable means is contacted with the carrier, wherein the means is preferably a labeled secondary antibody capable of binding to the antibody captured on the carrier,
c. optionally a means for removing any sample from the carrier and the detectable means, preferably by washing;
d. a detecting device for detecting the presence of the means for detecting and converting the results into an electrical signal, for example a fluorescence reader or a fluorescence microscope connected with a software capable of recognizing a pattern characteristic of a stained cell overexpressing a polypeptide comprising an antigen or at least one epitope thereof or a variant of the antigen or epitope thereof, wherein the antigen is NECAB1, in an image of the cell taken by the fluorescence reader or camera, and
optionally a means for receiving the electronical signal from the detecting device and determining if the level of the signal is indicative of an increased likelihood of having or developing a disease, by comparing with the patterns characteristic of wild type or non-stained cells, preferably by a mock-transfected cell or cells not positively stained by an antibody binding specifically to NECAB1 on the same carrier, or an input reference value obtained with samples from healthy subjects or by comparing the level of signal obtained with one sample with the level of signal obtained with a second sample obtained at a later time point, preferably at least one month later.

According to the present invention, a device for removing an antibody binding specifically to NECAB1 from blood, preferably serum of a patient suffering from a neurological autoimmune disease, wherein the device comprises a carrier with a solid phase on which a polypeptide comprising an antigen or at least one epitope thereof or a variant of the antigen or epitope thereof, wherein the antigen is NECAB1, is immobilized is provided as in an *ex vivo* method for removing an antibody binding specifically to NECAB1 from a patient. A device on which a polypeptide comprising an antigen or at least one epitope thereof or a variant of the antigen or epitope thereof, wherein the antigen is NECAB1, or a secondary antibody or protein capturing all IgG class antibodies, among them IgG class antibodies to NECAB1, may be used. Suitable methods are described in Eisei Noiri and Noria Hanafusa, The Concise Manual of Apharesis Therapy, Springer Tokyo, 2014. Hamilton, P., Kanigicherla, D., Hanumapura, P., Walz, L., Kramer, D., Fischer, M., Brenchley, P., and Mitra, S. (2018) J. Clin. Aph. 33(3), 283-290. Another method is disclosed in EP3477300.

In some embodiments, an antibody binding specifically to NECAB1 and/or its detection is used as a biomarker, preferably wherein the biomarker indicates the health status of a subject, in particular whether the subject has or is at risk of developing an autoimmune disease such as a neurological autoimmune disease and/or a cancer.

In a preferred embodiment, the methods, uses and products such as the polypeptide, the carrier or the kit, maybe used for diagnosing or for aiding in the diagnosis of a disease, preferably a neurological autoimmune disease and/or a cancer, as described herein.

The methods, uses and products described herein may also be used for the differential diagnosis of neurological diseases and/or cancer, e.g., for differentiating between a neurological autoimmune disease and a disease of different etiology but with similar symptoms such as an infectious neurological disease or an aseptic non-autoimmune neurological disease, for example between autoimmune encephalitis, including limbic encephalitis and brainstem encephalitis, and infectious and/or aseptic non-autoimmune encephalitis or meningitis, including encephalitis of infectious etiology, such as viral encephalitis, bacterial encephalitis, Tick-born encephalitis, Herpes encephalitis, CMV encephalitis, Toxoplasmic encephalitis, meningitis of infectious etiology, such as viral meningitis, bacterial meningitis, and aseptic non-autoimmune meningitis and encephalitis, such as Besnier-Boeck-Schaumann disease, Behcet's disease, Mollaret's meningitis.

In some embodiments, the term "one or more" comprises "at least one". In some embodiments, "one or more" and "at least one" are interchangeably. In some embodiments, the term "preferably" comprises "optionally". In some embodiments, "preferably" and "optionally" are interchangeably.

The present invention is further illustrated by the following non-limiting examples from which further features, embodiments, aspects and advantages of the present invention may be taken.
**Fig. 1** shows immunostaining on neuronal tissues. Diluted patient sera or CSF samples were incubated on rat cerebellum (not shown), rat hippocampus, or monkey cerebellum (not shown) and analyzed by immunofluorescence microscopy. Patient sera were subjected to immunoprecipitation experiments followed by SDS-PAGE. Specific bands were analyzed by mass spectrometry.
**Fig. 2** shows identification of target antigens. Patient serum was subjected to immunoprecipitation experiments followed by SDS-PAGE and Western Blot. Specific bands were analyzed by mass spectrometry.
**Fig. 3** shows the antigen functionality in recombinant cell based immunofluorescent assays (RC-IFA). Recombinant HEK293 cells expressing the respective antigen were acetone fixed and incubated with sera from index patients or control sera.
**Fig. 4** shows antigen functionality in Western Blot. Lysates of recombinant HEK293 cells expressing the respective antigen were analyzed by Western Blot with patient sera.
**Fig. 5** shows antigen functionality in Western Blot. Recombinant human His-NECAB1 purified from E.coli was analyzed by Western Blot with patient sera and Anti-His antibody.
**Fig. 6** shows autoantibody binding to His-C3b fused human NECAB1 fragments in Western Blot. All analyzed patient sera detect epitopes of NECAB1 in all three fragments. Patient samples do not react with other His-C3b fusion proteins (not shown).
**Fig. 7** shows peptide fragments of NECAB1 identified by mass spectrometry.

### Sequences

The present invention comprises a range of novel nucleic acid and amino acid sequences, more specifically
**SEQ ID NO1: human NECAB1** 
MEDSQETSPSSNNSSEELSSALHLSKGMSIFLDILRRADKNDDGKLSFEEFKAYFADGVLSG
EELHELFHTIDTHNTNNLDTEELCEYFSQHLGEYENVLAALEDLNLSILKAMGKTKKDYQEAS
NLEQFVTRFLLKETLNQLQSLQNSLECAMETTEEQTRQERQGPAKPEVLSIQWPGKRSSRR
VQRHNSFSPNSPQFNVSGPGLLEEDNQWMTQINRLQKLIDRLEKKDLKLEPPEEEIIEGNTK
SHIMLVQRQMSVIEEDLEEFQLALKHYVESASSQSGCLRISIQKLSNESRYMIYEFWENSSV
WNSHLQTNYSKTFQRSNVDFLETPELTSTMLVPASWWILNN
**SEQ ID NO2-SEQ ID NO10:** only in sequence listing
**SEQ ID NO11: sense NECAB1**
   ATACGTCTCACATGGAAGATTCCCAGGAGACATCG
**SEQ ID NO12: asense NECAB1**
   TATCGTCTCGTCGAGCTAGTTGTTCAGGATCCACCACG
**SEQ ID NO13: asense NECAB1-His without Stop-Codon**
   TATCGTCTCGTCGAGGTTGTTCAGGATCCACCACG
**SEQ ID NO14: asense NECAB1_aa110**
   ATACGTCTCCTCGATCATTAGATGGAAAGATTCAGGCCTTCAAG
**SEQ ID NO15: sense NECAB1_aa100**
   ATACGTCTCACATGCTAGCAGCACTTGAAGGCCTGAATC
**SEQ ID NO16: asense NECAB1_aa209**
   ATACGTCTCCTCGATCATTATTCTAATAAGCCTGGACCGCTGAC
**SEQ ID NO17: sense NECAB1_aa203**
   ATACGTCTCACATGAGCGGTCCAGGCTTATTAGAAGAAG
**SEQ ID NO18: amino acid residues 1-110 from human NECAB1** 
MEDSQETSPSSNNSSEELSSALHLSKGMSIFLDILRRADKNDDGKLSFEEFKAYFADGVLSG
EELHELFHTIDTHNTNNLDTEELCEYFSQHLGEYENVLAALEDLNLSI
**SEQ ID NO19: amino acid residues 100-209 from human NECAB1** 
LAALEDLNLSILKAMGKTKKDYQEASNLEQFVTRFLLKETLNQLQSLQNSLECAMETTEEQT
RQERQGPAKPEVLSIQWPGKRSSRRVQRHNSFSPNSPQFNVSGPGLLE
**SEQ ID NO20: amino acid residues 203-351 from human NECAB1** 
SGPGLLEEDNQWMTQINRLQKLIDRLEKKDLKLEPPEEEIIEGNTKSHIMLVQRQMSVIEEDL
EEFQLALKHYVESASSQSGCLRISIQKLSNESRYMIYEFWENSSVWNSHLQTNYSKTFQRS
NVDFLETPELTSTMLVPASWWILNN
**SEQ ID NO21: human NECAB1** 
MEDSQETSPSSNNSSEELSSALHLSKGMSIFLDILRRADKNDDGKLSFEEFKAYFADGVLSG
EELHELFHTIDTHNTNNLDTEELCEYFSQHLGEYENVLAALEGLNLSILKAMGKTKKDYQEAS
NLEQFVTRFLLKETLNQLQSLQNSLECAMETTEEQTRQERQGPAKPEVLSIQWPGKRSSRR
VQRHNSFSPNSPQFNVSGPGLLEEDNQWMTQINRLQKLIDRLEKKDLKLEPPEEEIIEGNTK
SHIMLVQRQMSVIEEDLEEFQLALKHYVESASSQSGCLRISIQKLSNESRYMIYEFWENSSV
WNSHLQTNYSKTFQRSNVDFLETPELTSTMLVPASWWILNN
**SEQ ID NO22: human NECAB1 (isoform)**

MLVQRQMSVIEEDLEEFQLALKHYVESASSQSGCLRISIQKLSNESRYMIYEFWENSSVWN
SHLQTNYSKTFQRSNVDFLETPELTSTMLVPASWWILNN

### Examples

### Summary

Methods: One index patient (P4) suffering from a neurological condition underwent serological investigation. For this purpose, patient serum was subjected to autoantibody screening by indirect immunofluorescence assay (IFA) with hippocampal and cerebellar tissue sections. Serum was analyzed in immunoprecipitation assays with brain tissue homogenates followed by mass spectrometry. The identified candidate antigen was recombinantly expressed in HEK293 cells and was applied in recombinant IFA.

Results: Neuronal tissue IFA screening of serum from P4 revealed an IgG reactivity pattern which corresponded to an antigen identified by immunoprecipitation and mass spectrometry: N-terminal EF-hand calcium-binding protein 1 (NECAB1). The serum IgG reactivity against the identified antigen was detected by recombinant cell based IFA (RC-IFA). Healthy control sera were also analyzed by RC-IFA. Index sample showed positive results while control sera were negative or showed low prevalence. Western blot reactivity of index sample with the corresponding recombinant antigens was analyzed. Clinical data from the index patient was available.

### Patients

Control collectives included sera from 100 healthy donors, 33 patients having chronic inflammatory demyelinating polyneuropathy, 30 patients having diabetic neuropathy, 30 patients having Guillain-Barré syndrome, 49 patients having SCLC but no PNS, and CSF from 89 patients having Alzheimer's disease, 15 patients having normal hydrocephalus, 20 patients having psychosis, 12 patients having dementia with Lewy bodies and 25 patients having frontotemporal dementia.

The index patient suffered from personality changes, cognitive impairment and affect disorder, mamma carcinoma, and limbic encephalitis. Further patients P1, P2, P3 and P5 suffered from cancer including mamma carcinoma and lung cancer, and from a neurological disease including neuronopathy, limbic encephalitis and cerebellar syndrome. A sixth patient (P6) having autoantibodies to NECAB1 was identified but clinical information was not available.

### Characterization of the patient's autoantibodies

Indirect immunofluorescence assays (IFA) of serum P1, P2, P3, and P4 using unfixed cryosections of rat hippocampus showed a distinct staining pattern (Figure 1).

### Immunoprecipitation

The immunoprecipitation was performed with 200 µl tissue homogenate of rat or monkey brain and 30 µl patient or control sera. Homogenates were centrifuged at 16000 x g for 10 minutes at 4°C. The sediment together with patient or control sera was resuspended in 500 µl lysis buffer (100 mmol/L tris-HCl pH 7.4, 150 mmol/L sodium chloride, 2.5 mmol/L EDTA, 0.5% (w/v) deoxycholate, 1% (w/v) Triton X-100 containing protease inhibitors) and rotated for 1 h at 4°C. The suspension was spun down at 16000 x g for 10 minutes at 4°C. The supernatants were then incubated with Protein G Dynabeads (ThermoFisher Scientific, Schwerte, Germany) at 4°C for 3 h to capture immunocomplexes. Beads were washed three times with PBS, and eluted with NuPage LDS sample buffer (ThermoFisher Scientific) containing 25 mmol/L dithiothreitol at 70°C for 10 min. Carbamidomethylation with 59 mM iodoacetamide (Bio-Rad, Hamburg, Germany) was performed prior to SDS-PAGE (NuPAGE, ThermoFisher Scientific). Separated proteins were visualized with Coomassie Brillant Blue (G-250), and identified by mass spectrometric analysis.

### Mass spectrometry

Visible protein bands were excised from Coomassie Brilliant Blue G-250 stained gels.

After destaining and tryptic digestion peptides were extracted, dried in SpeedVac and dissolved in water prior to nano-LC-MS/MS analysis.

Nano-LC-MS/MS measurements were performed with a Dionex Ultimate 3000 RSLC nanoLC coupled to a Bruker maXis II ETD QTOF via CaptiveSpray ionization source using otofControl 5.2 and Bruker Compass HyStar 5.1 (Bruker Daltonik GmbH, Bremen, Germany). Peptides were trapped on an Acclaim PepMap 100 C18 trap column and separated on an Acclaim PepMap 100 C18 analytical column (Thermo Scientific, Waltham, MA) using gradient elution. Spectra were recorded in positive mode with a scan speed of 4 Hz for precursor ions and a dynamic acquisition of 4 to 16 Hz for MS/MS spectra, in a mass range from 150 to 2200 m/z. Ions were fragmented by collision induced dissociation (CID) with nitrogen gas using a dynamic CID energy ranging from 23 to 65 eV depending on mass and charge state.

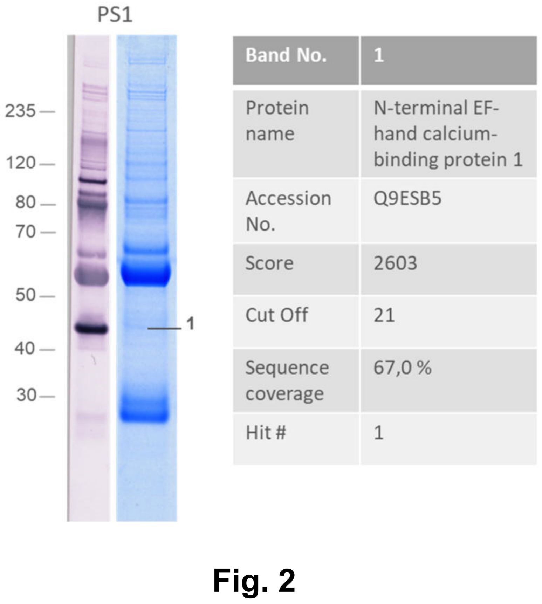

Spectra were recalibrated and processed with Bruker Compass DataAnalysis software 5.2. Database search against SwissProt limited to Mammalia was performed by Bruker BioPharma Compass software 3.1 using the Mascot search engine version 2.3 (Matrix Science, London, U.K.). To evaluate the protein hits, a significance threshold of p<0.05 was chosen. Peptides and proteins were accepted if their Mascot score exceeded 21 (Figure 2).

### Recombinant expression of antigens in HEK293 cells

The antigen human NECAB1 was cloned into mammalian expression vector pTriEx-1 (Merck, Darmstadt, Germany) and the corresponding protein was transiently expressed in the human cell line HEK293 following PEl-mediated transfection (Exgene 500), according to the manufacturer's instructions (Biomol GmbH, Hamburg, Germany). The sequence SEQ ID NO1, 11, 12, and 13 were used for cloning.

For the production of immunofluorescence substrates, the cells were grown on cover slides and acetone fixed two days after transfection.

For other purposes cells were harvested 5 days after transfection and lysed by high pressure. The lysates were stored in aliquots at -80 °C until further use.

### Recombinant expression of antigens in E.coli cells

The antigen human NECAB1 was cloned into bacterial expression vector pET24d (Merck, Darmstadt, Germany) and the corresponding protein was transiently expressed in *E.coli* cells. The sequences SEQ ID NO1, 11, 12, 14, 15, 16, and 17 were used for cloning of the NECAB1 fragments aa_1-110, aa-100-209, and aa_203-351.

### Indirect immunofluorescence assay (IFA)

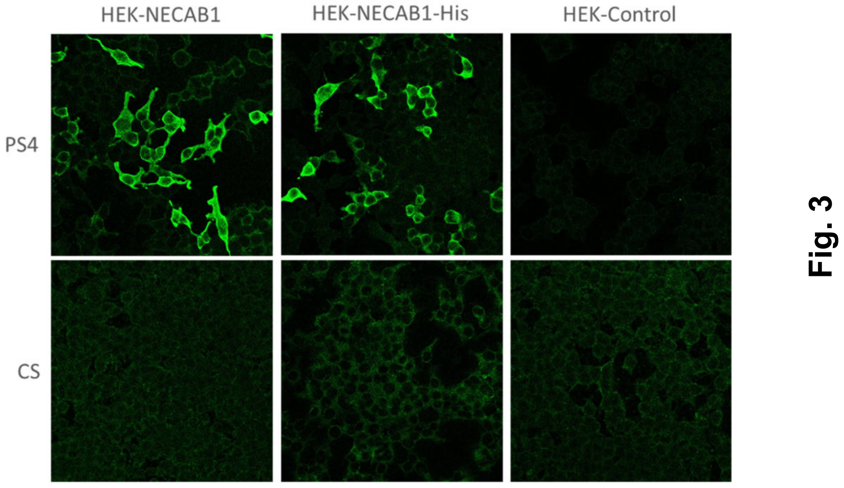

IFA was conducted using slides with biochip arrays of recombinant HEK293 cells expressing the identified antigen combined with control HEK293 cells transfected with an empty vector. Each biochip mosaic was incubated with 35 µL of 1:100 PBS-diluted sample at room temperature for 30 min, washed with PBS-Tween and immersed in PBS-Tween for 5 min. In the second step, fluorescein isothiocyanate (FITC)-labelled goat anti-human IgG (EUROIMMUN Medizinische Labordiagnostika AG, Lübeck) was applied and incubated at room temperature for 30 min. Slides were washed again with a flush of PBS-Tween and then immersed in PBS-Tween for 5 min. Slides were embedded in PBS-buffered, DABCO containing glycerol (approximately 10 µL per field) and examined by fluorescence microscopy. Positive and negative controls were included. Samples were classified as positive or negative based on fluorescence intensity of the transfected cells in direct comparison with non-transfected cells and control samples (Figure 3).

The association between the observed immunostaining pattern on CNS tissue and the identified antigen was confirmed by preincubation of patient sera with the respective recombinant antigen containing extract 1 h prior to incubation on tissue cryosections. For patient serum the specific immunostaining pattern was abolished by preincubation with the respective antigen NECAB1 containing extract but not with control extracts.

Results were evaluated by two independent observers using a EUROStar II microscope (EUROIMMUN Medizinische Labordiagnostika AG, Lübeck, Germany). Reagents were obtained from Merck, Darmstadt, Germany or Sigma-Aldrich, Heidelberg, Germany if not specified otherwise.

### Specificity of autoantibodies

100 healthy control sera and 93 sera from patients having neuropathy (n=33 chronic inflammatory demyelinating polyneuropathy, n=30 diabetic neuropathy and n=30 Guillain-Barré syndrome) were analyzed by IFA with HEK293 cells transfected with the respective recombinant antigen or mock transfected in a 1:100 dilution. None of 100 healthy control sera reacted in a similar way as the serum of the index patient. No reactivity was observed with sera from patients having SCLC but no PNS (n=49), nor with CSF from patients having Alzheimer's disease (n=89), patients having normal hydrocephalus (n=15), patients having psychosis (n=20), patients having dementia with Lewy bodies (n=12) and patients having frontotemporal dementia (n=25).

### Immunoblot

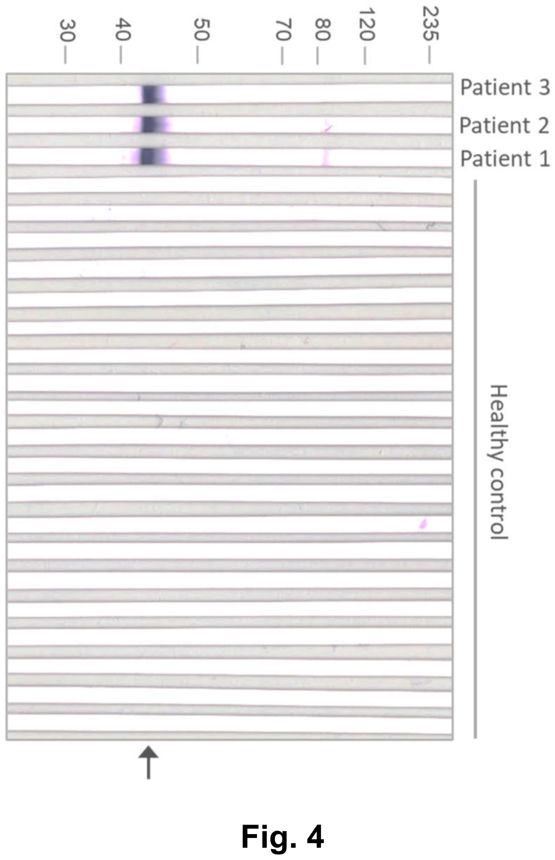

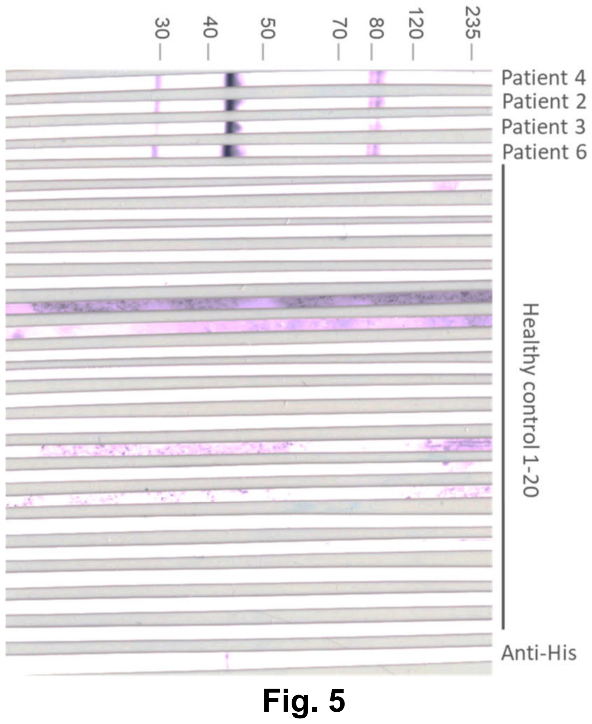

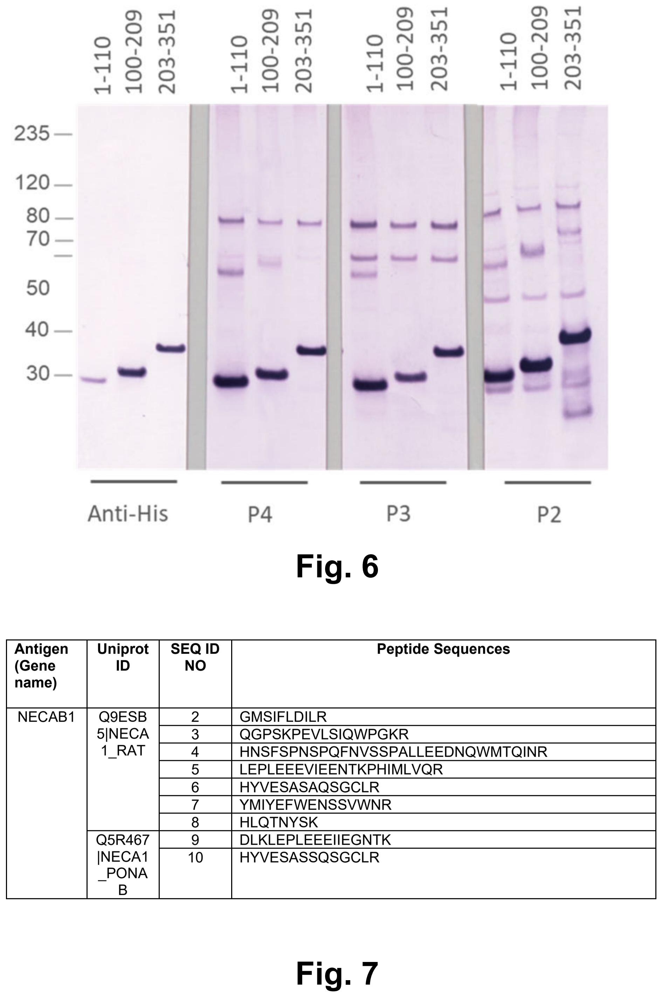

HEK-cell lysates containing the recombinantly expressed antigen, or purified NECAB1 recombinantly expressed in *E. coli*, or *E. coli* cell lysates expressing overlapping fragments (aa 1-110 (SEQ ID NO18), aa 100-209 (SEQ ID NO19), aa 203-351 (SEQ ID NO20)) of the identified antigen were incubated with NuPage LDS sample buffer (ThermoFisher Scientific, Schwerte, Germany) containing 25 mmol/L dithiothreitol at 70°C for 10 min, followed by SDS-PAGE (NuPAGE, ThermoFisher Scientific). Separated proteins were electrotransferred onto a nitrocellulose membrane by tank blotting with transfer buffer (ThermoFisher Scientific) according to the manufacturer's instructions. The membranes were blocked with Universal Blot Buffer plus (EUROIMMUN Medizinische Labordiagnostika AG, Lübeck, Germany) for 15 min and incubated with the patient or control sera (dilution 1:200) or anti-His (1:2000, 70796-3, Merck) in Universal Blot Buffer plus for 3 h, followed by three washing steps with Universal Blot Buffer (EUROIMMUN), a second incubation for 30 min with anti-human-IgG-AP (1:10, EUROIMMUN) or anti-mouse-IgG-AP (1:2000, Jackson immunoresearch, Suffolk, UK), three washing steps, and staining with NBT/BCIP substrate (EUROIMMUN). The results of the immunoblot experiments are shown in Figure 4, Figure 5 and Figure 6.

## Claims

1. A method comprising the step detecting in a sample a mammalian autoantibody binding specifically to NECAB1.

2. A method for isolating a mammalian autoantibody binding specifically to NECAB1, comprising the steps
a) immobilizing on a carrier a polypeptide comprising NECAB1 or at least one epitope thereof or a variant of NECAB1 or the epitope thereof,
b) contacting a sample comprising antibodies with the polypeptide under conditions compatible with formation of a complex, wherein said antibody binds to said polypeptide,
c) separating the complex formed in step a) from the sample,
d) optionally separating the antibody from the polypeptide, and
e) optionally detecting the antibody or complex.

3. A method comprising the steps
a) immobilizing a polypeptide comprising NECAB1 or at least one epitope thereof or a variant of NECAB1 or epitope thereof on a carrier,
b) contacting the carrier with a liquid comprising a mammalian autoantibody binding specifically to NECAB1, wherein the liquid does not comprise a sample from a subject to be diagnosed,
c) contacting the carrier with a means for detecting an immobilized antibody, and
d) detecting the presence of the antibody, preferably in quantitative manner.

4. A mammalian autoantibody binding specifically to NECAB1, preferably in a liquid comprising one or more, more preferably all from the group comprising an artificial buffer, a preservative and an artificial anticoagulant.

5. A kit comprising a polypeptide comprising NECAB1 or an epitope thereof or a variant of NECAB1 or the epitope thereof and a secondary antibody specifically binding to human immunoglobulins.

6. The kit according to claim 5, wherein the polypeptide and/or the secondary antibody comprises a label.

7. A use of
a) an antibody binding specifically to NECAB1; or
b) the combination of a polypeptide comprising NECAB1 or at least one epitope thereof or a variant of NECAB1 or the epitope thereof and a means for detecting or capturing an antibody, preferably IgG antibody; or
c) the kit according to any of claims 5 to 6
for identifying a patient having an increased risk of developing a disease associated with the presence of a mammalian autoantibody binding specifically to NECAB1.

8. A kit comprising a polypeptide comprising NECAB1 or at least one epitope thereof or a variant of NECAB1 or the epitope thereof and a carrier, wherein
a) the polypeptide is immobilized on a carrier, and the kit further comprises a secondary antibody specifically binding to human immunoglobulins,
b) the polypeptide and the carrier are configured for immobilizing the polypeptide on the surface of the carrier, preferably *via* an affinity tag and a ligand binding to the affinity tag, and the kit further comprises a secondary antibody specifically binding to human immunoglobulins,
c) the carrier is immobilized with a means for capturing an antibody, preferably a secondary antibody specifically binding to human immunoglobulins, and the kit further comprises a means for detecting an immobilized antibody, preferably the polypeptide comprising a detectable label, or
d) the carrier and a means for capturing an antibody, preferably a secondary antibody specifically binding to human immunoglobulins, are configured for immobilizing the means for capturing an antibody on the surface of the carrier, preferably via an affinity tag and a ligand binding to the affinity tag, and the kit further comprises a means for detecting an immobilized antibody, preferably the polypeptide comprising a detectable label,
and preferably one or more, more preferably all from the group consisting of a recombinant antibody binding to NECAB1, an isolated antibody binding to NECAB1, a chemical solution reactive with a detectable label, a positive control, a negative control, a water-tight vessel for incubating a sample with a carrier or reagent, a wash buffer, and a calibrator, preferably a set of calibrators.

9. A use of a polypeptide comprising NECAB1 or at least one epitope thereof or a variant of NECAB1 or the epitope thereof or an autoantibody binding specifically to NECAB1 or a recombinant antibody binding specifically to NECAB1 for the manufacture of an analytical kit or medical device, for diagnosing or aiding in the diagnosis of a disease associated with the presence of a mammalian autoantibody binding specifically to NECAB1.

10. A use of a mammalian autoantibody binding specifically to NECAB1 or a recombinant antibody binding specifically to NECAB1, which is preferably recognized by secondary antibodies to human immunoglobulins, as a positive control for the detection of a mammalian autoantibody binding specifically to NECAB1 in a sample.

11. An *ex vivo* method for removing a mammalian autoantibody binding specifically to NECAB1 from blood, preferably serum of a patient.

12. The method according to any one of claims 2-3, the kit according to any one of claims 5-6, the use according to claim 7 or 9, or the kit according to claim 8, wherein the polypeptide is a recombinant, isolated and/or purified polypeptide.

13. The method according to any one of claims 1-3 and 11-12, the use according to any one of claims 7, 9, 10 and 12, or the kit according to any one of claims 5-6, 8 and 12, wherein the autoantibody is a human autoantibody or the sample is a mammalian, preferably human sample comprising a representative set of autoantibodies, preferably selected from the group comprising whole blood, plasma, serum, cerebrospinal fluid and saliva.

14. The method according to any one of claims 1-3 and 11-13, the use according to any one of claims 7, 9, 10 and 12-13, or the kit according to any one of claims 5-6, 8 and 12-13, wherein the autoantibody or complex is detected using a detection method selected from the group consisting of immunodiffusion, electrophoresis, light scattering immunoassays, agglutination, labeled immunoassays such as those from the group comprising radiolabeled immunoassay, enzyme immunoassays, more preferably ELISA, chemiluminescence immunoassays, preferably electrochemiluminescence immunoassay, and immunofluorescence, preferably indirect immunofluorescence.

15. The method according to any one of claims 1-3 and 11-14, the use according to any one of claims 7, 9, 10 and 12-14, or the kit according to any one of claims 8 and 12-14, wherein the carrier is selected from the group consisting of a glass slide, preferably for microscopy, a biochip, a microtiter plate, a lateral flow device, a test strip, a membrane, preferably a line blot, a chromatography column and a bead, preferably a magnetic or fluorescent bead.
